# EUROPÄISCHE PATENTSCHRIFT

(11) **EP 0 915 087 B1**
(45) Veröffentlichungstag und Bekanntmachung des Hinweises auf die Patenterteilung: **02.04.2003**
(21) Anmeldenummer: 98120913.3
(22) Anmeldetag: 04.11.1998
(51) Int. Cl.: C07C 311/07, C07D 333/66, C07D 213/42, A61K 31/18, A61K 31/38, A61K 31/44

(54) **Sulfonamid-substituierte anellierte 5-Ring-Verbindungen, ihre Verwendung als Medikament sowie sie enthaltende pharmazeutische Zubereitungen**
Sulfonamide substituted fused five-membered ring compounds, their use as medicaments as well as pharmaceutical compositions containing them
Composés cycliques à cinq chaînons annelés portant un groupe sulfonamide, leur utilisation comme médicaments ainsi que compositions pharmaceutiques les contenant

(30) Priorität: 10.11.1997 DE 19749453
(43) Veröffentlichungstag der Anmeldung: 12.05.1999
(73) Patentinhaber: Aventis Pharma Deutschland GmbH, 65929 Frankfurt am Main (DE)
(72) Erfinder: Brendel, Joachim Dr., 61118 Bad Vilbel (DE); Gerlach, Uwe Dr., 65795 Hattersheim (DE); Lang, Hans Jochen Dr., 65719 Hofheim (DE); Weidmann, Klaus Dr., 61476 Kronberg (DE)

(56) Entgegenhaltungen:
- EP-A- 0 253 321
- GB-A- 2 135 999

## Beschreibung

Die Erfindung betrifft Verbindungen der Formel I, 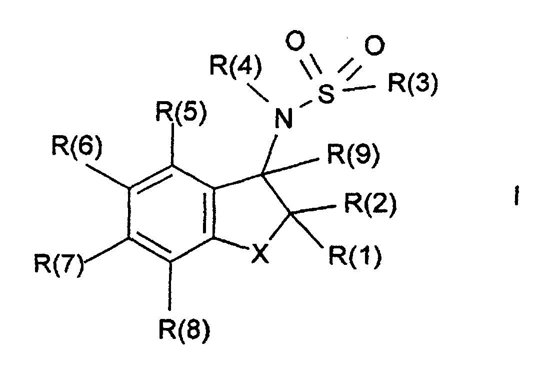 worin R(1), R(2), R(3), R(4), R(5), R(6), R(7), R(8), R(9) und X die im folgenden angegebenen Bedeutungen haben, ihre Herstellung und ihre Verwendung, insbesondere in Arzneimitteln. Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acylaminochroman-Derivate intensiv bearbeitet. Prominentester Vertreter dieser Klasse ist das Cromakalim der Formel A (J. Med. Chem. 1986, 29, 2194). Daneben wurden aber auch homologe Verbindungen, wie das durch Ringverengung des Pyranrings abgeleitete Indan B (J. Med. Chem. 1991, 34, 919), synthetisiert und untersucht.

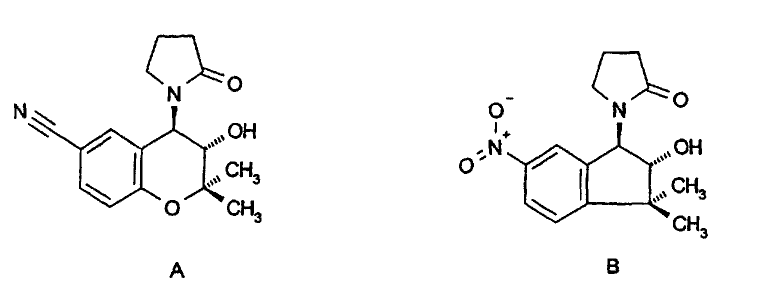

Bei Cromakalim und anderen verwandten 4-Acylaminochroman- bzw. 1-Acylaminoindan-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden können. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Von diesen Acylamino-Derivaten unterscheiden sich die erfindungsgemäßen Verbindungen der Formel I strukturell unter anderem durch den Ersatz der Acylaminogruppe durch eine Sulfonylaminofunktion. Während Cromakalim (Formel A) und analoge Acylaminoverbindungen (z.B. Verbindung B) als Öffner ATP-sensitiver K⁺-Kanäle wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺(cAMP) -Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP) -Kanals bzw. des I_{Ks}-Kanals entwickeln die erfindungsgemäßen Verbindungen im lebenden Organismus pharmakologische Wirkungen von hoher therapeutischer Verwertbarkeit.

Neben den oben genannten Cromakalim- bzw. Acylaminochroman-Derivaten werden in der Literatur auch Verbindungen mit 4-Sulfonylaminochroman-Struktur beschrieben, die sich aber sowohl in der Struktur als auch in der biologischen Wirkung deutlich von den erfindungsgemäßen Verbindungen der Formel I unterscheiden. So werden in der EP-A-315 009 Chromanderivate mit 4-Phenylsulfonylaminostruktur beschrieben, die sich durch antithrombotische und antiallergische Eigenschaften auszeichnen. In der EP-A-389 861 und der JP 01294677 werden 3-Hydroxychroman- bzw. Chromen-Derivate mit einer cyclischen 4-Sulfonylaminogruppe beschrieben (z.B. Verbindung C), die über eine Aktivierung des K⁺(ATP)-Kanals als Antihypertensiva wirken sollen. In der EP-A-370 901 werden 3-Hydroxychroman- bzw. Chromen-Derivate mit einer 4-Sulfonylaminogruppe, wobei die restliche Valenz des N-Atoms ein Wasserstoffatom trägt, beschrieben, die über ZNS-Wirkungen verfügen. Weitere 4-Sulfonylaminochroman-Derivate werden in Bioorg. Med. Chem. Lett. 4 (1994), 769 - 773: "N-sulfonamides of benzopyranrelated potassium channel openers: conversion of glyburyde insensitive smooth muscle relaxants to potent smooth muscle contractors" sowie in FEBS Letters 396 (1996), 271-275: "Specific blockade of slowly activating I_{sK} channels by chromanols ..." und Pflügers Arch. - Eur. J. Physiol. 429 (1995), 517-530: "A new class of inhibitors of cAMP-mediated Cl- secretion in rabbit colon, acting by the reduction of cAMP-activated K⁺ conductance" beschrieben.

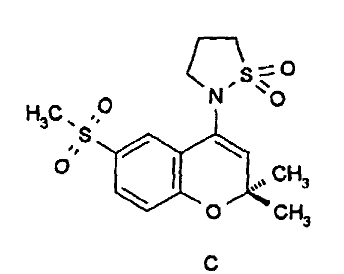

Die EP 0 253 321 beschreibt Verbindungen welche in 2-Position einen Sulfonylaminorest tragen, während bei den erfindungsgemäßen Verbindungen dieser Rest in 1-Position stehen muss. Weiterhin ist dieser Rest nach der Offenbarung der EP 0 253 321 (dort R1) zwingend Phenyl, während nach der Erfindung in der analogen Position (R3) kein aromatischer Rest stehen darf. Die Verbindungen nach der EP 0 253 321 haben antithrombotische Wirkung.

Die GB 2 135 999 beschreibt Indane, die in der Position R1 eine Fülle unterschiedlicher Funktionen tragen können. Im Unterschied dazu ist erfindungsgemäß an dieser Stelle zwingend ein Alkylsulfonylaminosubstituent erforderlich.

Die vorliegende Erfindung betrifft Verbindungen der Formel I, 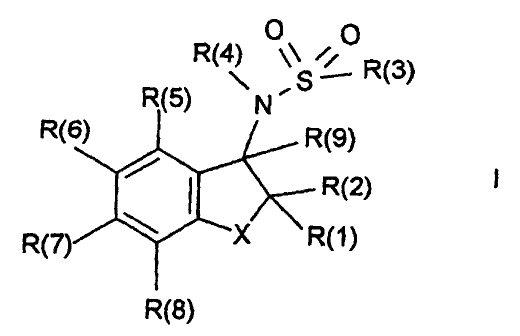 worin bedeuten:
R(1) und R(2)
unabhängig voneinander Wasserstoff, CF₃, Alkyl mit 1, 2, 3, 4, 5 oder 6 C-Atomen;
oder R(2)
-OR(10a), wenn X -CR(22)R(23)- bedeutet;
R(10a) Wasserstoff, Acetyl oder Alkyl mit 1, 2 oder 3 C-Atomen;
R(3) R(10b)-CₙH₂ₙ-NR(11)- oder R(10b)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-; R(12a) Wasserstoff, Methyl oder Ethyl;
R(10b) Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10b) und R(11)
gemeinsam eine Bindung, sofern n größer als 2 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(14)- oder-CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, -C_{z}H_{2z}OR(12c);
R(12c) Wasserstoff, Methyl oder Ethyl;
z 2 oder 3;
r Null, 1, 2, 3, 4, 5, 6, 7, 8, 9, 10, 11, 12, 13, 14, 15, 16, 17, 18, 19 oder 20;
R(5), R(6), R(7) und R(8)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(10c)-oder -CONR(10c)-, wobei die Verknüpfung mit dem Grundgerüst jeweils über das links stehende Atom erfolgt;
R(10c) Wasserstoff oder Alkyl mit 1, 2 oder 3 C-Atomen;
s Null, 1, 2, 3, 4, 5 oder 6;
R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Wasserstoff oder Alkyl mit 1, 2 oder 3 C-Atomen;
R(9) Wasserstoff
X -CR(22)R(23)-, -O-, -NR(24)-, -S-, -SO-, -SO₂-;
R(22) und R(23)
unabhängig voneinander Wasserstoff, CF₃, Alkyl mit 1, 2, 3, 4, 5, oder 6 C-Atomen;
R(24) Wasserstoff, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
sowie ihre physiologisch verträglichen Salze.

Bevorzugt sind Verbindungen der Formel I, worin bedeuten:
R(1) Wasserstoff;
R(2) Wasserstoff oder -OR(10a);
R(10a) Wasserstoff, Acetyl oder Alkyl mit 1, 2 oder 3 C-Atomen;
R(3) R(10b)-CₙH₂ₙ-NR(11)- oder R(10b)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10b) Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10b) und R(11)
gemeinsam eine Bindung, sofern n größer als 2 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen,
-C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, -C_{z}H_{2z}OR(12c);
R(12c) Wasserstoff, Methyl oder Ethyl;
z 2 oder 3;
r Null, 1, 2, 3, 4, 5, 6, 7, 8, 9, 10, 11, 12, 13, 14, 15, 16, 17, 18, 19 oder 20;
R(5), R(6), R(7) und R(8)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(10c) oder -CONR(10c)-, wobei die Verknüpfung mit dem Grundgerüst jeweils über das links stehende Atom erfolgt;
R(10c) Wasserstoff oder Alkyl mit 1, 2 oder 3 C-Atomen;
s Null, 1, 2, 3, 4, 5 oder 6;
R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Wasserstoff oder Alkyl mit 1, 2 oder 3 C-Atomen;
R(9) Wasserstoff;
X -CR(22)R(23)-;
R(22) und R(23)
unabhängig voneinander Wasserstoff, CF₃, Alkyl mit 1, 2, 3, 4, 5 oder 6 C-Atomen;
sowie ihre physiologisch verträglichen Salze.

Besonders bevorzugt sind Verbindungen der Formel I, worin bedeuten:
R(1) Wasserstoff;
R(2) Wasserstoff oder -OR(10a);
R(10a) Wasserstoff, Acetyl oder Alkyl mit 1, 2 oder 3 C-Atomen;
R(3) R(10b)-CₙH₂ₙ-,
R(10b) Methyl, Cycloalkyl mit 3, 4, 5 oder 6 C-Atomen, CF₃, C₂F₅ oder C₃F₇;
n Null, 1, 2, 3, 4 oder 5;
R(4) R(13)-CᵣH₂ᵣ,
wobei eine CH₂-Gruppe der Gruppe CᵣH₂ᵣ ersetzt sein kann durch -O-, -CO-, -CO-O-, -O-CO-, -NR(14)- oder -CONR(14)-;
R(14) Wasserstoff, Alkyl mit 1, 2 oder 3 C-Atomen,
-C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5 oder 6 C-Atomen,
-NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff oder Alkyl mit 1, 2 oder 3 C-Atomen;
r Null, 1, 2, 3, 4, 5, 6, 7, 8, 9 oder 10;
R(5), R(6), R(7) und R(8)
unabhängig voneinander Wasserstoff, F, Cl, Br, Alkyl mit 1, 2, 3, 4 oder 5 C-Atomen, Cycloalkyl mit 3, 4, 5 oder 6 C-Atomen, -CN, -CF₃, -C₂F₅, -C₃F₇, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-;
s Null, 1, 2, 3, 4, 5 oder 6;
R(18) Wasserstoff, Methyl, CF₃, C₂F₅, C₃F₇, Cycloalkyl mit 3, 4, 5 oder 6 C-Atomen, -OR(21), -COOR(21), -NR(15a)R(16a),
-CONR(15a)R(16a), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Alkyl mit 1, 2 oder 3 C-Atomen;
R(9) Wasserstoff
X -CR(22)R(23)-;
R(22) und R(23)
unabhängig voneinander Wasserstoff, CF₃, Alkyl mit 1, 2 oder 3 C-Atomen;
sowie ihre physiologisch verträglichen Salze.

Ganz besonders bevorzugt sind Verbindungen der Formel I, worin bedeuten:
R(1) Wasserstoff;
R(2) Wasserstoff oder -OR(10a);
R(10a) Wasserstoff oder Methyl;
R(3) R(10b)-CₙH₂ₙ-,
R(10b) Methyl;
n Null, 1 oder 2;
R(4) R(13)-CᵣH₂ᵣ,
wobei eine CH₂-Gruppe der Gruppe CᵣH₂ᵣ ersetzt sein kann durch -O-, -CO-O-, oder -CONR(14)-;
R(14) Wasserstoff, Alkyl mit 1, 2 oder 3 C-Atomen,
-C_{y}H_{2y}-OR(12b);
R(12b) Wasserstoff, Methyl oder Ethyl;
y 2 oder 3;
R(13) CH₃, CF₃ oder ein N-haltiger Heterocyclus mit 1, 2, 3, 4, 5, 6, 7, 8 oder 9 C-Atomen,
wobei der N-haltige Heterocyclus unsubstituiert ist oder substituiert mit 1 oder 2 Substituenten, ausgewählt aus der Gruppe bestehend aus F, Cl, Br, CF₃, NO₂, CN, Methyl und Methoxy;
r Null, 1, 2, 3, 4, 5, 6, 7 oder 8;
R(5), R(7) und R(8)
Wasserstoff;
R(6) Wasserstoff, F, Cl, Br, Alkyl mit 1, 2, 3, 4 oder 5 C-Atomen, Cycloalkyl mit 3, 4, 5 oder 6 C-Atomen, -CN, -CF₃, -C₂F₅, -C₃F₇, -NO₂, -Y-CₛH₂ₛ-R(18), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, Methyl und Methoxy;
Y -O-;
s 1, 2, 3, 4, 5 oder 6;
R(18) Wasserstoff, CF₃, -OR(21), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, Methyl und Methoxy;
R(21) Alkyl mit 1, 2 oder 3 C-Atomen;
R(9) Wasserstoff;
X -CR(22)R(23)-;
R(22) und R(23)
unabhängig voneinander CF₃ oder Methyl;
sowie ihre physiologisch verträglichen Salze.

Speziell bevorzugt sind Verbindungen der Formel I, worin bedeuten:
R(1) Wasserstoff;
R(2) Wasserstoff;
R(3) Methyl oder Ethyl;
R(4) R(13)-CᵣH₂ᵣ,
wobei eine CH₂-Gruppe der Gruppe CᵣH₂ᵣ ersetzt sein kann durch -O-;
R(13) CH₃ oder CF₃;
r Null, 1, 2, 3, 4, 5 oder 6;
R(5), R(7) und R(8)
Wasserstoff;
R(6) F, Cl, Br, Alkyl mit 1, 2, 3, 4 oder 5 C-Atomen, -CN, -CF₃, -NO₂, -Y-CₛH₂ₛ-R(18) oder Phenyl
das unsubstituiert ist oder substituiert mit 1 oder 2 Substituenten, ausgewählt aus der Gruppe bestehend aus F, Cl, Br, CF₃, NO₂, CN, Methyl und Methoxy;
Y -O-;
s 1, 2, 3, 4, 5 oder 6;
R(18) Wasserstoff, CF₃ oder Phenyl,
das unsubstituiert ist oder substituiert mit 1 oder 2 Substituenten, ausgewählt aus der Gruppe bestehend aus F, Cl, Br, CF₃, NO₂, CN, Methyl und Methoxy;
R(9) Wasserstoff;
X -CR(22)R(23)-;
R(22) und R(23)
Methyl;
sowie ihre physiologisch verträglichen Salze.

Speziell bevorzugt sind auch Verbindungen der Formel I, worin bedeuten:
R(1) Wasserstoff;
R(2) OH;
R(3) Methyl oder Ethyl;
R(4) R(13)-CᵣH₂ᵣ,
wobei eine CH₂-Gruppe der Gruppe CᵣH₂ᵣ ersetzt sein kann durch -O-;
R(13) CH₃ oder CF₃;
r Null, 1, 2, 3, 4, 5 oder 6;
R(5), R(7) und R(8)
Wasserstoff;
R(6) F, Cl, Br, Alkyl mit 1, 2, 3, 4 oder 5 C-Atomen, -CN, -CF₃, -NO₂, -Y-CₛH₂ₛ-R(18) oder Phenyl
das unsubstituiert ist oder substituiert mit 1 oder 2 Substituenten, ausgewählt aus der Gruppe bestehend aus F, Cl, Br, CF₃, NO₂, CN, Methyl und Methoxy;
Y -O-;
s 1,2, 3, 4, 5 oder 6;
R(18) Wasserstoff, CF₃ oder Phenyl,
das unsubstituiert ist oder substituiert mit 1 oder 2 Substituenten, ausgewählt aus der Gruppe bestehend aus F, Cl, Br, CF₃, NO₂, CN, Methyl und Methoxy;
R(9) Wasserstoff;
X -CR(22)R(23)-;
R(22) und R(23)
Methyl;
sowie ihre physiologisch verträglichen Salze.

Alkylreste und Alkylenreste können geradkettig oder verzweigt sein. Dies gilt auch für die Alkylenreste der Formeln CᵣH₂ᵣ, CₙH₂ₙ und CₛH₂ₛ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Als N-haltige Heterocyclen mit 1, 2, 3, 4, 5, 6, 7, 8 oder 9 C-Atomen gelten insbesondere die aromatischen Systeme 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder-5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Thienyl steht sowohl für 2- als auch 3-Thienyl. Furyl steht für 2- und 3-Furyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position.

Entsprechendes gilt sinngemäß analog auch für die N-haltigen Heterocylen oder den Thiophenrest.

Bei Disubstitution eines Restes können die Substituenten gleich oder verschieden sein.

Bedeuten R(2) und R(9) gemeinsam eine Bindung so liegt ein 3H-Indengrundgerüst vor, sofern X für CR(22)R(23) steht, bzw. ein Indol, Benzofuran oder Benzothiophen, sofern X für NR(24), O oder S steht. Bedeuten R(10b) und R(11) gemeinsam eine Bindung, so steht die Gruppe R(10)-CₙH₂ₙ-NR(11)- bevorzugt für einen über ein Stickstoffatom gebundenen Stickstoffheterocyclus. Wenn R(10) und R(11) gemeinsam eine Bindung bedeuten und die Gruppe R(10)-CₙH₂ₙ-NR(11)- für einen über ein Stickstoffatom gebundenen Stickstoffheterocyclus steht, ist dieser Stickstoffheterocyclus bevorzugt ein 4-Ring oder ein größerer Ring als ein 4-Ring, z. B. ein 5-Ring, 6-Ring oder 7-Ring.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zu der Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Racematen durch die Erfindung umfaßt. Bei Vorliegen einer cis/trans-Isomerie gehören sowohl die cis-Form als auch die transForm und Gemische dieser Formen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar. So erhält man beispielsweise eine Verbindung der Formel I, indem man
a) eine Verbindung der Formel II, 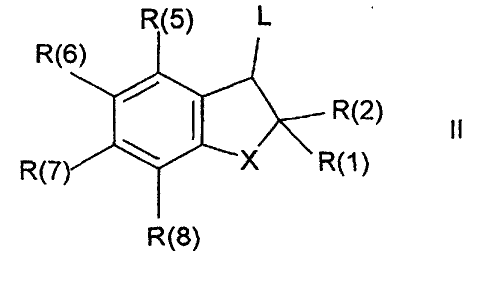 worin R(1), R(2), R(5), R(6), R(7), R(8) und X die oben angegebenen Bedeutungen besitzen und L eine nucleofuge Fluchtgruppe, insbesondere Cl, Br, I, Methansulfonyloxy, Trifluormethansulfonyloxy oder p-Toluolsulfonyloxy, bedeutet, in an sich bekannter Weise umsetzt mit einem Sulfonamid oder dessen Salz der Formel III, 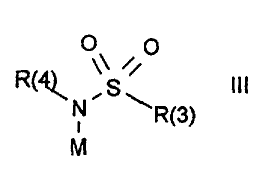 worin R(3) und R(4) die oben angegebenen Bedeutungen besitzen und M für Wasserstoff oder vorzugsweise für ein Metalläquivalent, besonders bevorzugt für Lithium, Natrium oder Kalium steht;
   oder daß man
b) eine Verbindung der Formel IV 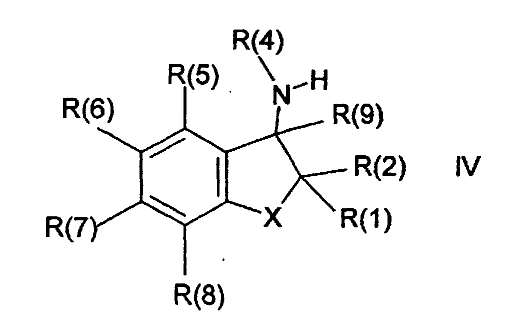 worin R(1), R(2), R(4), R(5), R(6), R(7), R(8), R(9) und X die oben angegebenen Bedeutungen besitzen, mit einem Sulfonsäure-Derivat der Formel V 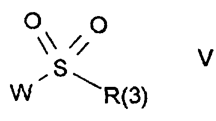 umsetzt, worin R(3) die oben angegebenen Bedeutungen besitzt und W eine nucleofuge Fluchtgruppe, wie z. B. Fluor, Brom, 1-Imidazolyl, insbesondere aber Chlor, bedeutet;
   oder daß man
c) eine Verbindung der Formel VI 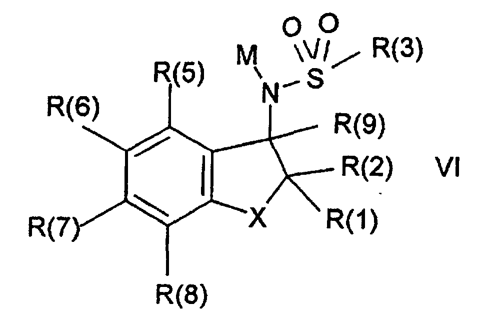 worin R(1), R(2), R(3), R(5), R(6), R(7), R(8), R(9), X und M die oben angegebenen Bedeutungen besitzen, in an sich bekannter Weise im Sinne einer Alkylierungsreaktion mit einem Alkylierungsmittel der Formel VII umsetzt,

   R(4)-L VII

   worin R(4) und L die oben angegebenen Bedeutungen besitzen;
   oder daß man
d) in einer Verbindung der Formel I, 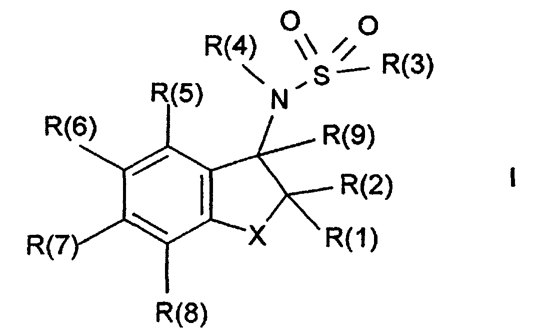 worin R(1) bis R(9) und X die oben angegebenen Bedeutungen besitzen, in mindestens einer der Positionen R(5), R(6), R(7) und R(8) eine elektrophile Substitutionsreaktion durchführt, sofern diese Position Wasserstoff bedeutet;
   oder daß man
e) eine Verbindung der Formel VIII 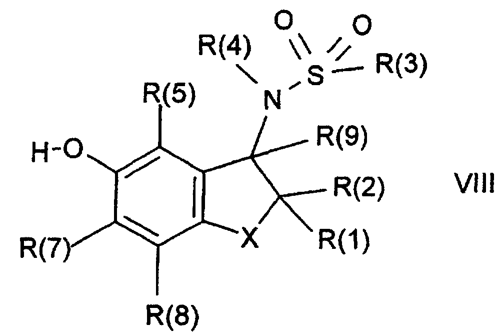 worin R(1), R(2), R(3), R(4), R(5), R(7), R(8), R(9) und X die oben angegebenen Bedeutungen besitzen, mit einer Verbindung der Formel R(18)-CₛH₂ₛ-L, worin R(18), s und L die oben angegebenen Bedeutungen besitzen, umsetzt im Sinne einer Alkylierungsreaktion; oder daß man
f) eine Verbindung der Formel IX, 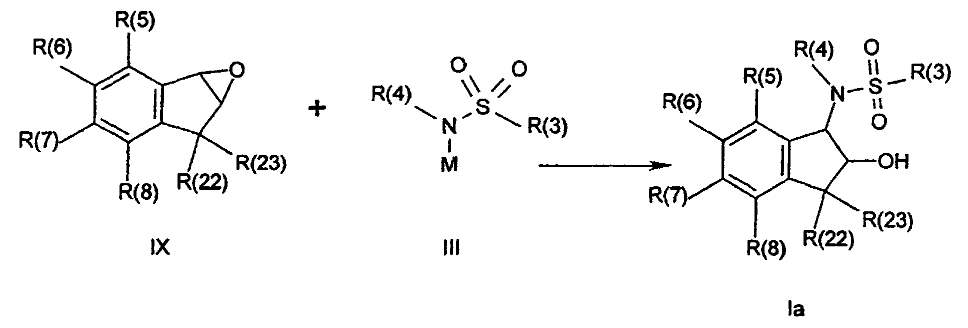 worin R(5), R(6), R(7), R(8), R(22) und R(23) die oben angegebenen Bedeutungen besitzen, mit einem Sulfonamid der Formel III, worin R(3) und R(4) die oben angegebenen Bedeutungen haben und M für Wasserstoff oder ein Metalläquivalent, bevorzugt für Lithium, Natrium oder Kalium, oder M vorteilhaft auch für einen Trialkylsilylrest, z.B. einen Trimethylsilylrest, steht, umsetzt zu einem Hydroxyindan der Formel la;
   oder daß man
g) eine Verbindung der Formel Ia mit einem Alkylierungsmittel der Formel R(10a)-L oder einem Acylierungsmittel der Formel CH₃COL bzw. einem Anhydrid der Formel (CH₃CO)₂O, worin R(10a) und L die oben angegeben Bedeutungen besitzen mit Ausnahme von Wasserstoff, umsetzt in an sich bekannter Weise im Sinne einer Alkylierungs- bzw. Acylierungsreaktion zu einer Verbindung der Formel Ib, worin R(3), R(4), R(5), R(6), R(7), R(8), R(10a), R(22) und R(23) die oben angegebenen Bedeutungen haben. 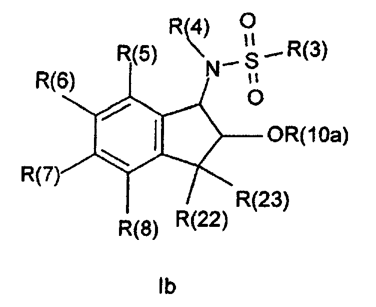 oder daß man
h) eine Verbindung der Formel la, 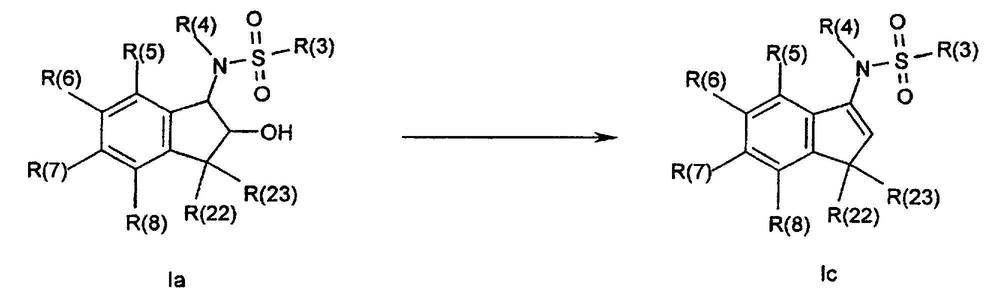 worin R(3), R(4), R(5), R(6), R(7), R(8), R(22) und R(23) die oben angegebenen Bedeutungen haben, im Sinne einer Eliminierungsreaktion zu einer Verbindung der Formel Ic, worin R(3), R(4), R(5), R(6), R(7), R(8), R(22) und R(23) die oben angegebenen Bedeutungen haben, umwandelt;

Die Verfahrensweise a) entspricht der nucleophilen Substitution einer Abgangsgruppe in einem reaktiven Bicyclus der Formel II durch ein Sulfonamid bzw. eines seiner Salze der Formel III. Wegen der höheren Nucleophilie und höheren Reaktivität eines in der Salzform vorliegenden Sulfonamids ist bei Verwendung eines freien Sulfonamids (Formel III, M = H) bevorzugt, aus diesem zunächst durch Einwirkung einer Base ein Sulfonamidsalz (Formel III, M = Metallkation) zu erzeugen. Setzt man ein freies Sulfonamid (Formel III, M = H) ein, so kann die Deprotonierung des Sulfonamids zum Salz in situ erfolgen. Bevorzugt werden solche Basen verwandt, die selbst nicht oder nur wenig alkyliert werden, wie z. B. Natriumcarbonat, Kaliumcarbonat, sterisch stark gehinderte Amine, z. B. Dicyclohexylamin, N,N-Dicyclohexyl-ethylamin, oder andere starke Stickstoffbasen mit geringer Nucleophilie, beispielsweise DBU (1,8-Diazabicyclo[5.4.0]undec-7-en) oder N,N',N'''-Triisopropylguanidin. Es können allerdings auch andere üblich verwendete Basen für die Reaktion eingesetzt werden, wie Kalium-tert-butylat, Natriummethylat, Alkalihydrogencarbonate, Alkalihydroxide, wie beispielsweise LiOH, NaOH oder KOH, oder Erdalkalihydroxide, wie beispielsweise Ca(OH)₂.

Vorzugsweise arbeitet man in einem Lösungsmittel, besonders bevorzugt in polaren organischen Lösungsmitteln wie z.B. Dimethylformamid (DMF), Dimethylacetamid (DMA), Dimethylsulfoxid (DMSO), Tetramethylharnstoff (TMU), Hexamethylphosphorsäuretriamid (HMPT), Tetrahydrofuran (THF), Dimethoxyethan (DME) oder anderen Ethem, oder beispielsweise auch in einem Kohlenwasserstoff wie Toluol oder in einem halogenierten Kohlenwasserstoff wie Chloroform oder Methylenchlorid. Es kann aber auch in polaren protischen Lösungsmitteln gearbeitet werden, wie z. B. in Wasser, Methanol, Ethanol oder Isopropanol. Die Reaktion wird bevorzugt in einem Temperaturbereich von -10 bis +140 °C, besonders bevorzugt im Bereich von 20 bis 100 °C durchgeführt. In günstiger Weise kann Verfahrensweise a) auch unter den Bedingungen einer Phasentransferkatalyse durchgeführt werden.

Die Verbindungen der Formel II gewinnt man nach literaturbekannten Methoden, beispielsweise aus den entsprechenden Alkoholen (Formel II, L = -OH) durch Einwirkung von Halogenwasserstoff HL (L = Cl, Br, I) oder durch Einwirkung eines anorganischen Säurehalogenids (POCl₃, PCl₃, PCl₅, SOCl₂, SOBr₂) oder durch radikalische Halogenierung der entsprechenden Derivate der Formel II in denen L Wasserstoff bedeutet mit radikalisch aktivierbaren Halogenierungsmitteln wie N-Bromsuccinimid (NBS) oder SO₂Cl₂ (Sulfurylchlorid) in Gegenwart eines Radikalkettenstarters wie energiereichem Licht des sichtbaren oder ultravioletten Wellenbereiches oder durch Verwendung eines chemischen Radikalstarters wie Azodiisobutyronitril.

Verfahrensweise b)
beschreibt die an sich bekannte und häufig angewandte Reaktion einer reaktiven Sulfonylverbindung der Formel V, insbesondere einer Chlorsulfonylverbindung (W = Cl), mit einem Amino-Derivat der Formel IV zum entsprechenden Sulfonamid-Derivat der Formel I.

Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.

Die Amine der Formel IV erhält man in literaturbekannter Weise bevorzugt aus den entsprechenden Carbonylverbindungen der Formel XX, 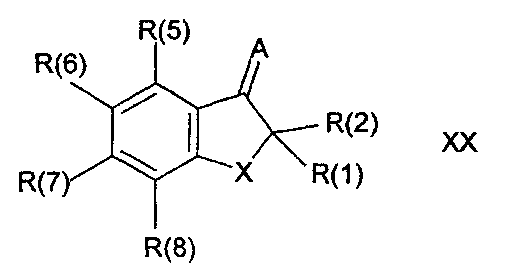 worin R(1), R(2), R(5), R(6), R(7), R(8) und X die oben angegebenen Bedeutungen besitzen und A Sauerstoff bedeutet, entweder mit Ammoniak oder einem Amin der Formel XXI,

R(4)-NH₂ XXI

in der R(4) die angegebenen Bedeutungen hat, unter reduktiven Bedingungen oder reduktiven katalytischen Bedingungen, vorzugsweise bei höherer Temperatur und im Autoklaven. Dabei werden primär durch Kondensationsreaktion der Ketone der Formel XX (A = Sauerstoff) und der Amine der Formel XXI in situ Schiff-Basen der Formel XX, in der A für R(4)-N= steht, gebildet, die unmittelbar, d. h. ohne vorherige Isolierung, reduktiv in die Amine der Formel IV überführt werden können. Es können aber auch die bei der Kondensationsreaktion intermediär aus den Verbindungen der Formel XX und XXI entstehenden Schiff-Basen (Formel XX, A steht für R(4)-N=) nach literaturbekannten Methoden hergestellt und zunächst isoliert werden, um sie dann in einem separaten Schritt mit einem geeigneten Reduktionsmittel, wie z. B. NaBH₄, LiAlH₄, NaBH3CN oder durch katalytische Hydrierung in Gegenwart von z. B. Raney-Nickel oder einem Edelmetall wie z. B. Palladium, in die Verbindungen der Formel IV umzuwandeln.

Die Verbindungen der Formel IV, in der R(4) für Wasserstoff steht, können vorteilhaft in literaturbekannter Weise auch durch Reduktion von Oximen oder Oximethern (Formel XX, A steht für =N-OR, R = H oder Alkyl) oder Hydrazonen (Formel XX, A steht für =N-NR₂, R z. B. = H oder Alkyl) erhalten werden, z. B. unter Verwendung eines komplexen Metallhydrids oder durch katalytische Hydrierung. Die dafür erforderlichen Oxime und Hydrazone werden vorzugsweise in an sich bekannter Weise aus den Ketonen der Formel XX (A = Sauerstoff) mit Hydrazin oder einem seiner Derivate oder beispielsweise mit Hydroxylamin-Hydrochlorid unter wasserabspaltenden Bedingungen hergestellt.

Besonders vorteilhaft können die Verbindungen der Formel IV, in der R(4) für Wasserstoff steht, auch durch Aminierung von Ketonen der Formel XX (A = Sauerstoff) mit einer geeigneten Ammoniumverbindung, z. B. Ammoniumacetat, in Gegenwart eines geeigneten Reduktionsmittels, wie z.B. NaCNBH₃, erhalten werden (J. Am. Chem. Soc. 93, 1971, 2897).

Die Ketone der Formel XX (A = Sauerstoff) sind entweder bekannt oder können analog beschriebenen Methoden hergestellt werden. Einige geeignete Ketone der Formel XX, bei denen X CR(22)R(23) bedeutet, sind z.B. beschrieben in J. Org. Chem. 19, 1954, 305 oder Org. Prep. Proc. Int. 10, 1978, 123. Ketone der Formel XX, bei denen X Sauerstoff bedeutet, sind z.B. beschrieben in J. Org. Chem. 26, 1961, 4758 oder Monatsh. Chem. 125, 1994, 971.

Alternativ können die Amino-Derivate der Formel IV auch in an sich literaturbekannter Weise durch Reaktion der reaktiven Verbindungen der Formel II mit R(1), R(2), R(5), R(6), R(7), R(8), X und L in der angegebenen Bedeutung, entweder mit Ammoniak oder einem Amin der Formel XXI, mit R(4) in der angegebenen Bedeutung, erhalten werden.

Verfahrensweise c)
repräsentiert die an sich bekannte Alkylierungsreaktion eines Sulfonamids bzw. eines seiner Salze VI mit einem Alkylierungsmittel der Formel VII. Entsprechend der Reaktionsanalogie mit Verfahrensweise a) gelten für Verfahrensweise c) die bereits ausführlich unter Verfahrensweise a) beschriebenen Reaktionsbedingungen.

Die Herstellung der Sulfonamid-Derivate VI (mit M=H) und deren Vorprodukte wurden bereits bei Verfahrensweise b) beschrieben, wobei R(4) dann jeweils Wasserstoff bedeutet. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

Verfahrensweise d)
beschreibt die weitere chemische Umwandlung von erfindungsgemäßen Verbindungen der Formel I in andere Verbindungen der Formel I durch elektrophile Substitutionsreaktionen in einer oder in mehreren der mit R(5) bis R(8) bezeichneten Positionen, die jeweils Wasserstoff bedeuten.

Bevorzugte Substitutionsreaktionen sind
1. die aromatische Nitrierung zur Einführung einer oder mehrerer Nitrogruppen, die in nachfolgenden Reaktionen teilweise oder alle zu Aminogruppen reduziert werden können. Die Aminogruppen können wiederum in nachfolgenden Reaktionen in andere Gruppen umgewandelt werden, beispielsweise in einer Sandmeyerreaktion, z. B. zur Einführung von Cyanogruppen;
2. die aromatische Halogenierung insbesondere zur Einführung von Chlor, Brom oder Jod;
3. die Chlorsulfonierung, z.B. durch Einwirkung von Chlorsulfonsäure, zur Einführung einer Chlorsulfonylgruppe, die in nachfolgenden Reaktionen in andere Gruppen umgewandelt werden kann, z. B. in eine Sulfonamidgruppe;
4. die Friedel-Crafts-Acylierungsreaktion zur Einführung eines Acylrestes oder eines Sulfonylrestes durch Einwirkung der entsprechenden Säurechloride in Gegenwart einer Lewis-Säure als Friedel-Crafts-Katalysator, vorzugsweise in Gegenwart von wasserfreiem Aluminiumchlorid.

Verfahrensweise e)
beschreibt die Alkylierung eines Phenols der Formel VIII mit einem Alkylierungsmittel der Formel R(18)-CₛH₂ₛ-L. Hierzu wird das Phenol zunächst durch Einwirkung einer geeigneten Base, wie z.B. Natriumhydrid, in ein Phenolatsalz überführt, das dann in einem geeigneten polaren Lösungsmittel, wie z.B. Dimethylformamid oder Dimethylacetamid, bei Temperaturen zwischen 20 und 150°C mit dem Alkylierungsmittel umgesetzt wird. Die Deprotonierung des Alkohols zum Salz kann auch in situ erfolgen, wobei dann bevorzugt Basen eingesetzt werden, die selbst nicht alkyliert werden, wie z.B. Kaliumcarbonat. Als weitere geeignete Basen und Lösungsmittel können auch die bereits unter Verfahrensweise a) genannten verwendet werden.
Die Phenole der Formel VIII erhält man nach den oben unter a) bis c) bzw. weiter unten unter f) bis h) beschriebenen Methoden, wobei dann jedoch R(6) jeweils OH bzw. OR (R=geeignete Schutzgruppe, z.B. Benzyl) bedeutet und im letzteren Fall noch eine anschließende Abspaltung der Schutzgruppe erfolgt.

Verfahrensweise f)
entspricht der nucleophilen Öffnung eines Epoxids der Formel IX durch ein Sulfonamid bzw. eines seiner Salze der Formel III. Bei Verwendung eines freien Sulfonamids (Formel III, M = H) ist bevorzugt, aus diesem zunächst durch Einwirkung einer Base ein Sulfonamidsalz (Formel III, M = Metallkation) zu erzeugen, wobei die Deprotonierung des Sulfonamids zum Salz in situ erfolgen kann. Hierzu können die bereits unter Verfahrensweise a) aufgeführten Basen verwendet werden.

Die Base kann in stöchiometrischer Menge oder auch katalytisch eingesetzt werden. Als besonders vorteilhaft hat sich die Verwendung des freien Sulfonamids in Gegenwart einer kleinen Menge, z.B. 20%, des entsprechenden Sulfonamidnatriumsalzes erwiesen, das man aus dem Sulfonamid z.B. durch Zugabe von 0,2 Moläquivalenten Natriumhydrid erhalten kann.

Die Reaktion wird vorzugsweise in einem Lösungsmittel durchgeführt, besonders bevorzugt in polaren organischen Lösungsmitteln wie z. B. Dimethylformamid (DMF), Dimethylacetamid (DMA), Dimethylsulfoxid (DMSO), Tetramethylharnstoff (TMU), Hexamethylphosphorsäuretriamid (HMPT), Tetrahydrofuran (THF) oder Dimethoxyethan (DME). Die Reaktion wird bevorzugt in einem Temperaturbereich von -10 bis +140 °C, besonders bevorzugt im Bereich von 20 bis 100 °C durchgeführt.

Eine andere bevorzugte Verfahrensweise zur Durchführung dieser Reaktion beinhaltet die Verwendung von Sulfonamidderivaten der Formel III, bei denen M für einen Trialkylsilyl-, z.B. einen Trimethylsilylrest steht. Hierbei ist es vorteilhaft, die Reaktion in Gegenwart eines Fluorids, z.B. Tetrabutylammoniumfluorid, durchzuführen.

Die Epoxide der Formel IX erhält man nach literaturbekannten Methoden aus den entsprechenden Olefinen der Formel XXII, 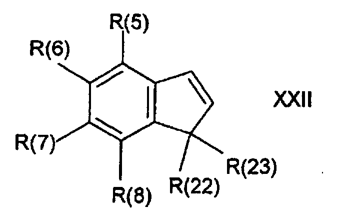 worin R(5), R(6), R(7), R(8), R(22) und R(23) die oben angegebenen Bedeutungen besitzen, z. B. durch Einwirkung eines geeigneten anorganischen oder organischen Peroxids, wie beispielsweise H₂O₂ oder m-Chlorperbenzoesäure, oder durch basenkatalysierte Cyclisierung des entsprechenden Bromhydrins, das aus XXII z. B. durch Umsetzung mit N-Bromsuccinimid und Wasser erhalten werden kann. Die Epoxide der Formel IX können aus Olefinen der Formel XXII auch in optisch reiner Form durch Oxidation in Gegenwart des chiralen Jacobsen-Katalysators erhalten werden, wie z. B. in Tetrahedron Letters 32, 1991, 5055 beschrieben. Die Olefine der Formel XXII lassen sich erhalten aus den Ketonen der Formel XX (A = O, X = CR(22)R(23)) durch Reduktion der Carbonylgruppe zu einer OH-Funktion, z.B. mit Natriumborhydrid, und anschließende säurekatalysierte Eliminierung, z.B. durch Erhitzen mit p-Toluolsulfonsäure in Toluol.

Verfahrensweise g)
beschreibt die Umwandlung von erfindungsgemäßen Verbindungen der Formel la zu anderen erfindungsgemäßen Verbindungen der Formel Ib durch Alkylierung bzw. Acylierung der 2-Hydroxygruppe. Für die Alkylierung wird der Alkohol zunächst durch Einwirkung einer geeigneten Base, wie z.B. Natriumhydrid, in ein Alkoholatsalz überführt, das dann in einem geeigneten polaren Lösungsmittel, wie z.B. Dimethylformamid, bei Temperaturen zwischen 20 und 150°C mit dem Alkylierungsmittel der Formel R(10a)-L umgesetzt wird. Die Deprotonierung des Alkohols zum Salz kann auch in situ erfolgen, wobei dann bevorzugt Basen eingesetzt werden, die selbst nicht alkyliert werden, wie z.B. Kaliumcarbonat. Als weitere geeignete Basen und Lösungsmittel können auch die bereits unter Verfahrensweise a) genannten verwendet werden. Die Acetylierung der Verbindungen der Formel la erfolgt bevorzugt durch Umsetzung mit Acetanhydrid in einem geeigneten polaren Lösungsmittel wie Pyridin oder Dimethylformamid und gegebenenfalls unter Zusatz eines Acylierungskatalysators wie z.B. Dimethylaminopyridin.

Verfahrensweise h)
beschreibt die Überführung eines 2-Hydroxyindans der Formel la in ein Inden der Formel Ic durch Eliminierung. Hierzu kann das 2-Hydroxyindan entweder direkt in Gegenwart einer Säure oder Base einer Wasserabspaltung unterworfen werden oder es kann zunächst eine Aktivierung der Hydroxygruppe erfolgen, z.B. durch Acetylierung mit Acetanhydrid (siehe Verfahrensweise g) oder Mesylierung mit Methansulfonsäurechlorid, woraufhin anschließend eine basenkatalysierte Eliminierung erfolgen kann, z.B. durch Erhitzen mit DBU (1,8-Diazabicyclo[5.4.0]-undec-7-en).

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Es wurde bereits gesagt, daß die Verbindungen der Formel I überraschenderweise eine starke und spezifische blockierende (schließende) Wirkung auf einen K⁺-Kanal haben, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine antidiarrhoische Wirkung aus und sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien, vor allem von Herzrhythmusstörungen, die durch Aktionspotential-Verlängerung behoben werden können. Sie können speziell angewandt werden zur Therapie und Prophylaxe von atrialer Fibrillation (Vorhofflimmern) und atrialem Flattern (Vorhofflattern) sowie zur Therapie und Prophylaxe von Reentry-Arrhythmien und zur Verhinderung des plötzlichen Herztodes infolge von Kammerflimmem.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht.

Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III-Antiarrhythmika blockieren überwiegend oder ausschließlich I_{Kr} (z.B. Dofetilid, d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44B-49B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T.J. Colatsky, C.H. Follmer und C.F. Starmer; "Channel Specificity in Antiarrhythmic Drug Action; Mechanism of potassium channel block and its role in suppressing and aggravating cardiac arrhythmias"; Circulation 82 (1990), 2235 - 2242; A.E. Busch, K. Malloy, W.J. Groh, M.D. Vamum, J.P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys. Res. Commun. 202 (1994), 265-270).

Darüber hinaus tragen die Verbindungen zu einer deutlichen Verbesserung der Herzinsuffizienz, insbesondere der Stauungsherzinsuffizienz (Congestive Heart Failure) bei, vorteilhafterweise in der Kombination mit kontraktionsfördernden (positiv inotropen) Wirkstoffen, z.B. Phosphordiesterasehemmem.

Trotz der therapeutisch nutzbaren Vorteile, die durch eine Blockade des I_{Ks} erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1:10). In der WO-A-95/14470 wird die Verwendung von Benzodiazepinen als selektive Blocker des I_{Ks} beansprucht. Weitere I_{Ks}-Blocker sind beschrieben in FEBS Letters 396 (1996), 271-275: "Specific blockade of slowly activating I_{sK} channels by chromanols ..." und Pflügers Arch. - Eur. J. Physiol. 429 (1995), 517-530: "A new class of inhibitors of cAMP-mediated Cl- secretion in rabbit colon, acting by the reduction of cAMP-activated K⁺ conductance".

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgem können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauf-Erkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H2-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- DMF: N,N-Dimethylformamid
- EE: Essigsäurethylester
- F.p.: Schmelzpunkt (Wenn nicht anders angegeben sind die Schmelzpunkte der ungereinigten Rohprodukte angegeben; die Schmelzpunkte der jeweiligen Reinsubstanzen können durchaus deutlich höher liegen)
- RT: Raumtemperatur
- THF: Tetrahydrofuran

### Beispiel 1: 3,3-Dimethyl-1-(N-methyl-N-methylsulfonyl)amino-indan

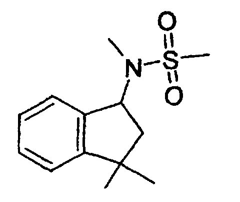

a) Eine Lösung von 25,0 g (0,156 mol) 3,3-Dimethyl-indan-1-on (Chem. Ber. 64, 1931, 1493) in 700 ml Methanol wurde mit 120,2 g (1,56 mol) Ammoniumacetat und 69,1 g (1,1 mol) Natriumcyanborhydrid 8 h bei 60°C gerührt. Die Reaktionsmischung wurde mit verdünnter Salzsäure auf pH<2 gestellt und einrotiert. Der Rückstand wurde in verdünnter Salzsäure aufgenommen, mit EE extrahiert und die wäßrige Phase dann mit Kaliumcarbonatlösung alkalisch gestellt und mit EE extrahiert. Nach Trocknen der organische Phase mit Magnesiumsulfat und Einengen im Vakuum wurden 10,3 g 1-Amino-3,3-dimethylindan erhalten.
b) 4,0 g (34,7 mmol) Methansulfonsäurechlorid wurden unter Eiskühlung zu einer Lösung von 5,1 g (31,6 mmol) 1-Amino-3,3-dimethylindan und 12,8 g (126,4 mmol) Triethylamin in 100 ml THF zugetropft. Nach 2 h bei RT wurden 100 ml Wasser zugegeben, die Reaktionsmischung bis auf ca. 50 ml eingeengt, mit 100 ml Wasser versetzt und der ausgefallene Niederschlag abgesaugt, mit Wasser nachgewaschen und im Vakuum getrocknet. Man erhielt 6,7 g 3,3-Dimethyl-1-methylsulfonylaminoindan; F.p. 117 - 119°C.
c) Eine Lösung von 1,0 g (4,2 mmol) 3,3-Dimethyl-1-methylsuifonylamino-indan in 16 ml DMF wurde zu einer Suspension von 0,15 g (5,1 mmol) 80proz. Natriumhydrid in 10 ml DMF getropft und es wurde 1 h bei RT nachgerührt. Dann wurden 0,59 g (4,2 mmol) Iodmethan zugegeben und bei RT 3 h nachgerührt. Nach Zugabe von 3 ml Wasser wurde die Reaktionsmischung im Vakuum komplett eingeengt, der Rückstand in EE und Wasser aufgenommen und die organische Phase mit verdünnter Salzsäure und gesättigter Natriumhydrogencarbonatlösung gewaschen. Nach Trocknen über Magnesiumsulfat und Einengen wurden 0,5 g 3,3-Dimethyl-1-(N-methyl-N-methylsulfonyl)amino-indan erhalten; F.p. 71 - 73°C.

### Beispiel 2: 3,3-Dimethyl-1-(N-ethylsulfonyl-N-methyl)amino-indan

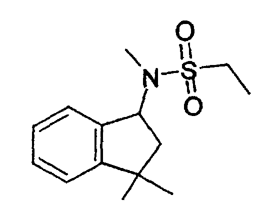

a) 4,44 g (34,7 mmol) Ethansulfonsäurechlorid wurden unter Eiskühlung zu einer Lösung von 5,1 g (31,6 mmol) 1-Amino-3,3-dimethylindan (Beispiel 1a) und 12,8 g (126 mmol) Triethylamin in 100 ml THF zugetropft. Nach 2 h bei RT wurde die Reaktionsmischung im Vakuum eingeengt und der Rückstand in Wasser und EE aufgenommen. Nach Trocknen der organischen Phase über Magnesiumsulfat und Einengen im Vakuum wurden 6,4 g 3,3-Dimethyl-1-ethylsulfonylamino-indan erhalten.
b) Eine Lösung von 1,0 g (4,2 mmol) 3,3-Dimethyl-1-ethylsulfonylamino-indan in 16 ml DMF wurde zu einer Suspension von 0,15 g (5,1 mmol) 80proz. Natriumhydrid in 10 ml DMF getropft und 1 h bei RT nachgerührt. Nach Zugabe von 0,59 g (4,2 mmol) lodmethan wurde weitere 3 h bei RT gerührt. Dann wurden 3 ml Wasser zugegeben, die Reaktionsmischung komplett einrotiert und der Rückstand in EE und Wasser aufgenommen. Die organische Phase wurde mit verdünnter Salzsäure und gesättigter Natriumhydrogencarbonatlösung gewaschen, mit Magnesiumsulfat getrocknet und im Vakuum eingeengt. Man erhielt 0,9 g 3,3-Dimethyl-1-(N-methyl-N-ethylsulfonyl)amino-indan als Öl.

### Beispiel 3: 3,3-Dimethyl-1-(N-hexyl-N-methylsulfonyl)amino-indan

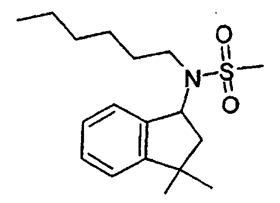

Eine Lösung von 1,0 g (4,2 mmol) 3,3-Dimethyl-1-methylsulfonylamino-indan (Beispiel 1b) in 16 ml DMF wurde zu einer Suspension von 0,15 g (5,1 mmol) 80proz. Natriumhydrid in 10 ml DMF getropft, und es wurde 1 bei RT nachgerührt. 0,89 g (4,2 mmol) 1-Iodhexan wurden zugegeben und 3 h bei RT gerührt. Dann wurden 3 ml Wasser zugegeben, die Reaktionsmischung komplett einrotiert und der Rückstand in EE und Wasser aufgenommen. Die organische Phase wurde mit verdünnter Salzsäure und gesättigter Natriumhydrogencarbonatlösung gewaschen, mit Magnesiumsulfat getrocknet und einrotiert. Man erhielt 1,1 g 3,3-Dimethyl-1-(Nhexyl-N-methylsulfonyl)amino-indan als Öl.
¹H-NMR (200 MHz, CDCl₃): δ [ppm] = 0.9 (t, 3H), 1.2 (s, 3H), 1.45 (s, 3H), 1.1 - 1.7 (m, 8H), 1.85 (dd, 1H), 2.25 (dd, 1H), 2.8 - 3.1 (m, 2H), 3.0 (s, 3H), 5.5 (t, 1H), 7.1 - 7.3 (m, 4H).

### Beispiel 4: 3,3-Dimethyl-1-(N-ethylsulfonyl-N-hexyl)amino-indan

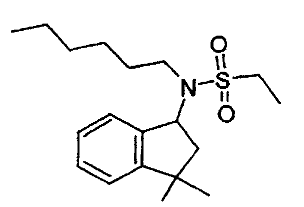

Eine Lösung von 1,0 g (4,0 mmol) 3,3-Dimethyl-1-ethylsulfonylamino-indan (Beispiel 2a) in 16 ml DMF wurde zu einer Suspension von 0,15 g (5,1 mmol) 80proz. Natriumhydrid in 10 ml DMF getropft und 1 h bei RT nachgerührt. 0,89 g (4,2 mmol) 1-lodhexan wurden zugegeben und 3 h bei RT gerührt. Dann wurden 3 ml Wasser zugegeben, die Reaktionsmischung komplett einrotiert und der Rückstand in EE und Wasser aufgenommen. Die organische Phase wurde mit verdünnter Salzsäure und gesättigter Natriumhydrogencarbonatlösung gewaschen, mit Magnesiumsulfat getrocknet und einrotiert. Man erhielt 1,1 g 3,3-Dimethyl-1-(N-ethylsulfonyl-N-hexyl)amino-indan als Öl.

### Beispiel 5: N-Butyl-N-(1,1-dioxo-2,3-dihydro-1H-benzo[b]thiophen-3-yl)-methansulfonamid

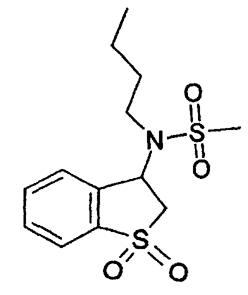

a) 9,0 g (54 mmol) Benzothiophen-1,1-dioxid und 7,9 g (108 mmol) n-Butylamin wurden in 130 ml Ethanol suspendiert und 3 h auf Rückflußtemperatur erhitzt. Die Reaktionsmischung wurde im Vakuum eingeengt und man erhielt 14,5 g Butyl-(1,1-dioxo-2,3-dihydro-1H-benzo[b]thiophen-3-yl)-amin als Öl.
b) 6,63 g (57 mmol) Methansulfonsäurechlorid wurden zu einer Lösung von 11,7 g (41 mmol) Butyl-(1,1-dioxo-2,3-dihydro-1H-benzo[b]thiophen-3-yl)-amin und 15,5 g (153 mmol) Triethylamin in 120 ml THF zugetropft und die Reaktionsmischung wurde über Nacht bei RT gerührt. Das THF wurde weitgehend abdestilliert, der Rückstand mit 250 ml Wasser verdünnt und der ausgefallene Niederschlag abgesaugt. Nach Trocknen im Vakuum wurden 13,8 g N-Butyl-N-(1,1-dioxo-2,3-dihydro-1H-benzo[b]thiophen-3-yl)methansulfonamid erhalten; F.p. 171 -173°C.

### Beispiel 6: N-Butyl-N-(6-nitro-1,1-dioxo-2,3-dihydro-1H-benzo[b]thiophen-3-yl)-methansulfonamid

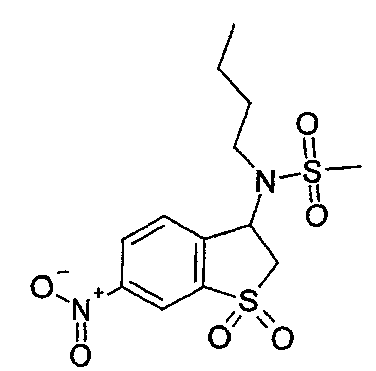

0,5 g (1,6 mmol) N-Butyl-N-(1,1-dioxo-2,3-dihydro-1H-benzo[b]thiophen-3-yl)methansulfonamid (Beispiel 5) wurden bei-10°C in 5 ml konzentrierter Schwefelsäure gelöst und mit 0,15 g (1,8 mmol) Natriumnitrat versetzt und 20 h bei RT gerührt. Die Reaktionsmischung wurde auf 50 ml Eiswasser gegossen, 30 min nachgerührt, abgesaugt, mit Wasser neutralgewaschen und im Vakuum getrocknet. Man erhielt 0,5 g N-Butyl-N-(6-nitro-1,1-dioxo-2,3-dihydro-1H-benzo[b]thiophen-3-yl)-methansulfonamid; F.p. 52 - 55°C.

### Beispiel 7: 5-Fluor-1-(N-methyl-N-methylsulfonyl)amino-indan

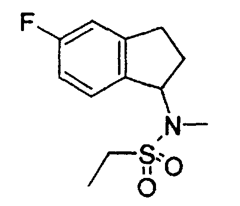

a) 4,5 g (30 mmol) 5-Fluor-1-indanon wurden in 20 ml Pyridin und 20 ml Ethanol mit 2,3 g (33 mmol) Hydroxylaminhydrochlorid 6 h auf 80°C erhitzt. Nach Abdestillieren der Lösungsmittel wurde der Rückstand mit Wasser verrührt und der ausgefallene Niederschlag abgesaugt und im Vakuum getrocknet. Man erhielt 4,8 g 5-Fluor-1-indanonoxim; F.p. 150 - 155°C.
b) 1,6 g 5-Fluor-1-indanonoxim wurden in 30 ml Methanol mit Raney-Nickel als Katalysator bei Normaldruck und RT hydriert. Nach Abfiltrieren des Katalysators und Abziehen des Lösungsmittels wurde das Produkt durch Ausfällen mit etherischer Salzsäure als Hydrochlorid isoliert. Man erhielt 0,6 g 5-Fluor-1-indanylaminhydrochlorid; F.p. 245 - 247°C.
c) 0,6 g 5-Fluor-1-indanylamin-hydrochlorid wurden analog Beispiel 2 a zu 0,6 g 5-Fluor-1-ethylsulfonylamino-indan umgesetzt.
d) Aus 0,6 g 5-Fluor-1-ethylsulfonylamino-indan wurden analog Beispiel 2 b 0,42 g 5-Fluor-1-(N-methyl-N-methylsulfonyl)amino-indan als Öl erhalten.

### Beispiel 8: R(+)-1-(N-Ethylsulfonyl-N-methyl)amino-indan

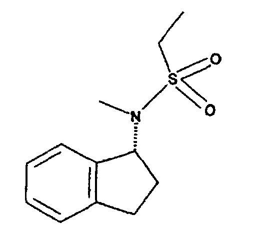

a) Aus 3 g R(-)-1-Aminoindan wurden analog Beispiel 2b 4,8 g R(+)-1-Ethylsulfonylaminoindan erhalten; F.p. 66 - 71°C, Drehwert +27,9°.
b) Aus 1,12 g R(+)-1-Ethylsulfonylaminoindan wurden analog Beispiel 2 b 0,87 g R(+)-1-(N-Ethylsulfonyl-N-methyl)amino-indan als Öl erhalten; Drehwert +15,9°.

### Beispiel 9: S(-)-1-(N-Ethylsulfonyl-N-methyl)amino-indan

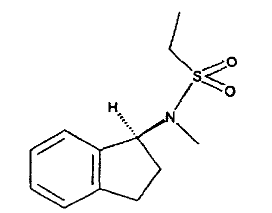

a) Aus 3 g S(+)-1-Aminoindan wurden analog Beispiel 2 b 5,1 g S(-)-1-Ethylsulfonylaminoindan erhalten; F.p. 64 - 68°C, Drehwert -29,5°.
b) Aus 1,12 g S(-)-1-Ethylsulfonylaminoindan wurden analog Beispiel 2 b 0,89 g S()-1-(N-Ethylsulfonyl-N-methyl)amino-indan als Öl erhalten; Drehwert +15,9°.

### Beispiel 10: 3,3-Dimethyl-6-nitro-1-(N-methyl-N-methylsulfonyl)amino-indan

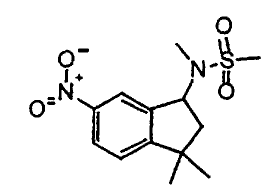

a) Durch Reduktion von 3,3-Dimethyl-6-nitro-indan-1-on (Org. Prep.Proced.Int. 10; 1978; 123) mit Natriumcyanborhydrid in Gegenwart von Ammoniumacetat analog Beispiel 1 a wurde 1-Amino-3,3-dimethyl-6-nitro-indan erhalten.
b) Aus 1,5 g 1-Amino-3,3-dimethyl-6-nitro-indan wurden durch Umsetzung mit Methansulfonsäurechlorid analog Beispiel 1 b 1,5 g 3,3-Dimethyl-6-nitro-1-methylsulfonylamino-indan erhalten; F.p. 145 - 147°C.
c) Eine Lösung von 0,15 g (0,5 mmol) 3,3-Dimethyl-6-nitro-1-methylsulfonylaminoindan, 0,12 g (0,5 mmol) tert-Butylimino-tris(dimethylamino)phosphoran (Phosphazenbase) und 0,08 g (0,55 mmol) lodmethan in 4 ml DMF wurde über Nacht bei RT gerührt. Nach Abdestillieren des Lösungsmittels wurde der Rückstand in EE aufgenommen und 2mal mit Wasser gewaschen. Nach Trocknen über Magnesiumsulfat und Einengen erhielt man 0,15 g 3,3-Dimethyl-6-nitro-1-(N-methyl-N-methylsulfonyl)amino-indan; F.p. 180 - 182°C.

### Beispiel 11: 3,3-Dimethyl-6-nitro-1-(N-butyl-N-methylsulfonyl)amino-indan

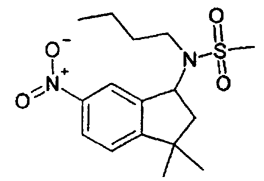

Die Verbindung wurde erhalten durch Alkylierung von 3,3-Dimethyl-6-nitro-1-methylsulfonylamino-indan mit 1-lodbutan analog Beispiel 10c. F.p. 110 - 112°C.

### Beispiel 12: 3,3-Dimethyl-6-nitro-1-[N-(4-pyridylmethyl)-N-methylsulfonyl]aminoindan

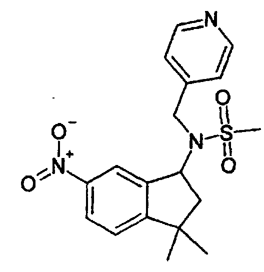

Die Verbindung wurde erhalten durch Alkylierung von 3,3-Dimethyl-6-nitro-1-methylsulfonylamino-indan mit 4-Pyridylmethylchlorid-hydrochlorid analog Beispiel 10c, wobei jedoch die doppelte molare Menge an Phosphazenbase eingesetzt wurde.

### Beispiel 13: 6-Butoxy-3,3-dimethyl-1-(N-methyl-N-methylsulfonyl)amino-indan

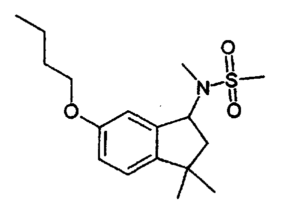

a) Zu einer Lösung von 5,6 g (0,19 mol) 80% Natriumhydrid in 250 ml DMF wurde unter Stickstoff eine Lösung von 28,5 g (0,16 mol) 6-Hydroxy-3,3-dimethyl-indan-1-on (J. Org. Chem. 19; 1954; 305) in 200 ml DMF zugetropft. Nach 1 h bei RT wurden 30,4 g (0,17 mol) 1-lodbutan zugegeben und es wurde 2 h bei RT gerührt. Nach Abdestillieren des Lösungsmittels im Vakuum wurde der Rückstand in EE und Wasser aufgenommen und die organische Phase wurde nacheinander mit verd. Salzsäure und verd. Natronlauge gewaschen. Man erhielt 31,3 g 6-Butoxy-3,3-dimethyl-indan-1-on.
b) Eine Lösung von 1,0 g (4,3 mmol) g 6-Butoxy-3,3-dimethyl-indan-1-on, 1,9 g (30 mmol) Natriumcyanborhydrid und 3,3 g (43 mmol) Ammoniumacetat in 30 ml Methanol wurde 5 h auf 60°C erhitzt. Nach Zugabe von etwas Wasser wurde das Methanol im Vakuum abgezogen und der Rückstand mit EE und Salzsäure versetzt. Die saure wäßrige Phase wurde abgetrennt, mit Natronlauge alkalisch gestellt und mit EE extrahiert. Man erhielt 0,4 g 1-Amino-6-butoxy-3,3-dimethyl-indan.
c) Aus 2,0 g 1-Amino-6-butoxy-3,3-dimethyl-indan wurden analog Beispiel 1 b 2,1 g 6-Butoxy-3,3-dimethyl-1-methylsulfonylamino-indan erhalten.
d) Durch Alkylierung von 0,5 g 6-Butoxy-3,3-dimethyl-1-methylsulfonylamino-indan mit lodmethan analog Beispiel 1c wurden 0,5 g 6-Butoxy-3,3-dimethyl-1-(N-methyl-N-methylsulfonyl)amino-indan als Öl erhalten.

### Beispiel 14: 6-Butoxy-3,3-dimethyl-1-(N-ethyl-N-methylsulfonyl)amino-indan

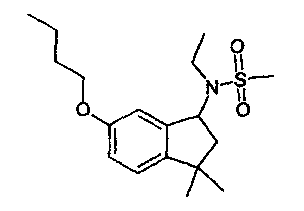

Die Verbindung wurde erhalten als Öl durch Alkylierung von 6-Butoxy-3,3-dimethyl-1-methylsulfonylamino-indan (Beispiel 13c) mit lodethan analog Beispiel 1 c. ¹H-NMR (CDCl₃): δ [ppm] = 1.0 (t, 3H), 1.2 (s, 3H), 1.2 (t, 3H), 1.4 (s, 3H), 1.3-1.8 (m, 4H), 1.9 (dd, 1H), 2.25 (dd, 1H), 3.0 (s, 3H), 3.1 (m, 2H), 3.95 (t, 2H), 5.45 (dd, 1H), 6.8 (m, 2H), 7.05 (d, 1H).

### Pharmakologische Untersuchungen

IsK-Kanäle aus Mensch, Ratte oder Meerschweinchen wurden in Xenopus-Oozyten exprimiert. Hierfür wurden zuerst Oozyten aus Xenopus Laevis isoliert und defollikuliert. Anschließend wurde in diese Oozyten in vitro synthetisierte IsKkodierende RNA injiziert. Nach 2 - 8 Tagen IsK-Proteinexpression wurden an den Oozyten mit der Zwei-Mikroelektroden Voltage-Clamp Technik IsK-Ströme gemessen. Die IsK-Kanäle wurden hierbei in der Regel mit 15 s dauernden Spannungssprüngen auf -10 mV aktiviert. Das Bad wurde mit einer Lösung der nachfolgenden Zusammensetzung durchspült: NaCI 96 mM, KCl 2 mM, CaCl₂ 1,8 mM, MgCl₂ 1 mM, HEPES 5 mM (titriert mit NaOH auf pH 7,5). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des IsK-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

### Literatur:

A.E. Busch, H.-G. Kopp, S. Waldegger, I. Samarzija, H. Süßbrich, G. Raber, K. Kunzelmann, J. P. Ruppersberg und F. Lang; "Inhibition of both exogenously expressed IsK and endogenous K+ channels in Xenopus oocytes by isosorbiddinitrate"; J. Physiol. 491 (1995), 735 - 741;
T. Takumi, H. Ohkubo und S. Nakanishi; "Cloning of a membrane protein that induces a slow voltage-gated potassium current"; Science 242 (1989), 1042 - 1045; M.D. Varnum, A.E. Busch, C.T. Bond, J. Maylie und J.P. Adelman; "The minK channel underlies the cardiac potassium current and mediates species-specific responses to protein kinase"; C. Proc. Natl. Acad. Sci. USA 90 (1993), 11528 - 11532.

Auf die beschriebene Weise unter Verwendung des humanen IsK-Proteins wurden für die erfindungsgemäßen Verbindungen folgende IC50-Werte bestimmt:

| Verbindung | IC- ₅₀ [µM] |
|---|---|
| Beispiel 2 | ca. 15 |
| Beispiel 3 | <10 |
| Beispiel 5 | >10 |
| Beispiel 11 | 2,7 |
| Beispiel 12 | ca. 2 |
| Beispiel 13 | 1,2 |
| Beispiel 14 | 0,44 |

## Patentansprüche

1. Verbindungen der Formel I, 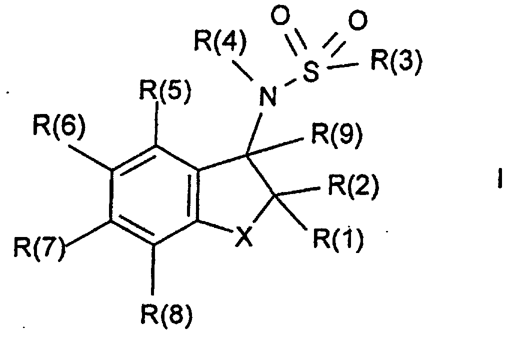 worin bedeuten:
R(1) und R(2)
unabhängig voneinander Wasserstoff, CF₃, Alkyl mit 1, 2, 3, 4, 5 oder 6 C-Atomen;
oder R(2)
-OR(10a), wenn X -CR(22)R(23)- bedeutet;
R(10a) Wasserstoff, Acetyl oder Alkyl mit 1, 2 oder 3 C-Atomen;
R(3) R(10b)-CₙH₂ₙ-NR(11)- oder R(10b)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10b) Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10b) und R(11)
gemeinsam eine Bindung, sofern n größer als 2 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, -C_{z}H_{2z}OR(12c);
R(12c) Wasserstoff, Methyl oder Ethyl;
z 2 oder 3;
r Null, 1, 2, 3, 4, 5, 6, 7, 8, 9, 10, 11, 12, 13, 14, 15, 16, 17, 18, 19 oder 20;
R(5), R(6), R(7) und R(8)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO₂-, -SO₂-, -O-SO₂-, -SO₂NR(10c)-oder -CONR(10c)-, wobei die Verknüpfung mit dem Grundgerüst jeweils über das links stehende Atom erfolgt;
R(10c) Wasserstoff oder Alkyl mit 1, 2 oder 3 C-Atomen;
s Null, 1, 2, 3, 4, 5 oder 6;
R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1,2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Wasserstoff oder Alkyl mit 1, 2 oder 3 C-Atomen;
R(9) Wasserstoff
X -CR(22)R(23)-, -O-, -NR(24)-, -S-, -SO-, -SO₂-;
R(22) und R(23)
unabhängig voneinander Wasserstoff, CF₃, Alkyl mit 1, 2, 3, 4, 5 oder 6 C-Atomen;
R(24) Wasserstoff, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
sowie ihre physiologisch verträglichen Salze.

2. Verbindungen der Formel I nach Anspruch 1, in der bedeuten:
R(1) Wasserstoff;
R(2) Wasserstoff oder -OR(10a);
R(10a) Wasserstoff, Acetyl oder Alkyl mit 1, 2 oder 3 C-Atomen;
R(3) R(10b)-CₙH₂ₙ-NR(11)- oder R(10b)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10b) Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10b) und R(11)
gemeinsam eine Bindung, sofern n größer als 2 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, -C_{z}H_{2z}OR(12c);
R(12c) Wasserstoff, Methyl oder Ethyl;
z 2 oder 3;
r Null, 1, 2, 3, 4, 5, 6, 7, 8, 9, 10, 11, 12, 13, 14, 15, 16, 17, 18, 19 oder 20;
R(5), R(6), R(7) und R(8)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(10c) oder -CONR(10c)-, wobei die Verknüpfung mit dem Grundgerüst jeweils über das links stehende Atom erfolgt;
R(10c) Wasserstoff oder Alkyl mit 1, 2 oder 3 C-Atomen;
s Null, 1, 2, 3, 4, 5 oder 6;
R(18) Wasserstoff, Methyl, CF₃, C₂F₅, C₃F₇, Cycloalkyl mit 3, 4, 5, 6, 7 oder 8 C-Atomen, -OR(21), -COOR(21), -NR(15a)R(16a), -CONR(15a)R(16a), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Wasserstoff oder Alkyl mit 1, 2 oder 3 C-Atomen;
R(9) Wasserstoff
X -CR(22)R(23)-;
R(22) und R(23)
unabhängig voneinander Wasserstoff, CF₃, Alkyl mit 1, 2, 3, 4, 5 oder 6 C-Atomen,
sowie ihre physiologisch verträglichen Salze.

3. Verbindungen der Formel I nach Ansprüchen 1 oder 2, in der bedeuten:
R(1) Wasserstoff;
R(2) Wasserstoff oder -OR(10a);
R(10a) Wasserstoff, Acetyl oder Alkyl mit 1, 2 oder 3 C-Atomen;
R(3) R(10b)-CₙH₂ₙ-,
R(10b) Methyl, Cycloalkyl mit 3, 4, 5 oder 6 C-Atomen, CF₃, C₂F₅ oder C₃F₇;
n Null, 1, 2, 3, 4 oder 5;
R(4) R(13)-CᵣH₂ᵣ,
wobei eine CH₂-Gruppe der Gruppe CᵣH₂ᵣ ersetzt sein kann durch -O-, -CO-, -CO-O-, -O-CO-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) CH₃, CF₃, C₂F₅, C₃F₇, Cycloalkyl mit 3, 4, 5 oder 6 C-Atomen, -NR(15)R(16), -CONR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2, 3 oder 4 C-Atomen;
oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff oder Alkyl mit 1, 2 oder 3 C-Atomen;
r Null, 1, 2, 3, 4, 5, 6, 7, 8, 9 oder 10;
R(5), R(6), R(7) und R(8)
unabhängig voneinander Wasserstoff, F, Cl, Br, Alkyl mit 1, 2, 3, 4 oder 5 C-Atomen, Cycloalkyl mit 3, 4, 5 oder 6 C-Atomen, -CN, -CF₃, -C₂F₅, -C₃F₇, -NO₂, -Y-CₛH₂ₛ-R(18), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-;
s Null, 1, 2, 3, 4, 5 oder 6;
R(18) Wasserstoff, Methyl, CF₃, C₂F₅, C₃F₇, Cycloalkyl mit 3, 4, 5 oder 6 C-Atomen, -OR(21), -COOR(21), -NR(15a)R(16a),
-CONR(15a)R(16a), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(15a) und R(16a)
unabhängig voneinander Wasserstoff oder Alkyl mit 1, 2, 3 oder 4 C-Atomen;
oder
R(15a) und R(16a)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(21) Alkyl mit 1, 2 oder 3 C-Atomen;
R(9) Wasserstoff
X -CR(22)R(23)-;
R(22) und R(23)
unabhängig voneinander Wasserstoff, CF₃, Alkyl mit 1, 2 oder 3 C-Atomen,
sowie ihre physiologisch verträglichen Salze.

4. Verbindungen der Formel I nach einem oder mehreren der Ansprüche 1 bis 3, in der bedeuten:
R(1) Wasserstoff;
R(2) Wasserstoff oder -OR(10a);
R(10a) Wasserstoff oder Methyl;
R(3) R(10b)-CₙH₂ₙ-,
R(10b) Methyl;
n Null, 1 oder 2;
R(4) R(13)-CᵣH₂ᵣ,
wobei eine CH₂-Gruppe der Gruppe CᵣH₂ᵣ ersetzt sein kann durch -O-, -CO-O-, oder -CONR(14)-;
R(14) Wasserstoff, Alkyl mit 1, 2 oder 3 C-Atomen, -C_{y}H_{2y}-OR(12b);
R(12b) Wasserstoff, Methyl oder Ethyl;
y 2 oder 3;
R(13) CH₃, CF₃ oder ein N-haltiger Heterocyclus mit 1, 2, 3, 4, 5, 6, 7, 8 oder 9 C-Atomen,
wobei der N-haltige Heterocyclus unsubstituiert ist oder substituiert mit 1 oder 2 Substituenten, ausgewählt aus der Gruppe bestehend aus F, Cl, Br, CF₃, NO₂, CN, Methyl oder Methoxy;
r Null, 1, 2, 3, 4, 5, 6, 7 oder 8;
R(5), R(7) und R(8)
Wasserstoff;
R(6) Wasserstoff, F, Cl, Br, Alkyl mit 1, 2, 3, 4 oder 5 C-Atomen, Cycloalkyl mit 3, 4, 5 oder 6 C-Atomen, -CN, -CF₃, -C₂F₅, -C₃F₇, -NO₂, -Y-CₛH₂ₛ-R(18), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, Methyl und Methoxy;
Y -O-;
s 1, 2, 3, 4, 5 oder 6;
R(18) Wasserstoff, CF₃, -OR(21), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, Methyl und Methoxy;
R(21) Alkyl mit 1, 2 oder 3 C-Atomen;
R(9) Wasserstoff
X -CR(22)R(23)-;
R(22) und R(23)
unabhängig voneinander CF₃ oder Methyl,
sowie ihre physiologisch verträglichen Salze.

5. Verbindungen der Formel I nach einem oder mehreren der Ansprüche 1 bis 4, in der bedeuten:
R(1) Wasserstoff;
R(2) Wasserstoff;
R(3) Methyl oder Ethyl;
R(4) R(13)-CᵣH₂ᵣ,
wobei eine CH₂-Gruppe der Gruppe CᵣH₂ᵣ ersetzt sein kann durch -O-;
R(13) CH₃ oder CF₃;
r Null, 1, 2, 3, 4, 5 oder 6;
R(5), R(7) und R(8)
Wasserstoff;
R(6) F, Cl, Br, Alkyl mit 1, 2, 3, 4 oder 5 C-Atomen, -CN, -CF₃, -NO₂, -Y-CₛH₂ₛ-R(18) oder Phenyl
das unsubstituiert ist oder substituiert mit 1 oder 2 Substituenten, ausgewählt aus der Gruppe bestehend aus F, Cl, Br, CF₃, NO₂, CN, Methyl und Methoxy;
Y -O-;
s 1, 2, 3, 4, 5 oder 6;
R(18) Wasserstoff, CF₃ oder Phenyl,
das unsubstituiert ist oder substituiert mit 1 oder 2 Substituenten, ausgewählt aus der Gruppe bestehend aus F, Cl, Br, CF₃, NO₂, CN, Methyl und Methoxy;
R(9) Wasserstoff;
X -CR(22)R(23)-;
R(22) und R(23)
Methyl,
sowie ihre physiologisch verträglichen Salze.

6. Verbindungen der Formel I nach einem oder mehreren der Ansprüche 1 bis 4, in der bedeuten:
R(1) Wasserstoff;
R(2) OH;
R(3) Methyl oder Ethyl;
R(4) R(13)-CᵣH₂ᵣ,
wobei eine CH₂-Gruppe der Gruppe CᵣH₂ᵣ ersetzt sein kann durch -O-;
R(13) CH₃ oder CF₃;
r Null, 1, 2, 3, 4, 5 oder 6;
R(5), R(7) und R(8)
Wasserstoff;
R(6) F, Cl, Br, Alkyl mit 1, 2, 3, 4 oder 5 C-Atomen, -CN, -CF₃, -NO₂, -Y-CₛH₂ₛ-R(18) oder Phenyl
das unsubstituiert ist oder substituiert mit 1 oder 2 Substituenten, ausgewählt aus der Gruppe bestehend aus F, Cl, Br, CF₃, NO₂, CN, Methyl und Methoxy;
Y -O-;
s 1, 2, 3, 4, 5 oder 6;
R(18) Wasserstoff, CF₃ oder Phenyl,
das unsubstituiert ist oder substituiert mit 1 oder 2 Substituenten, ausgewählt aus der Gruppe bestehend aus F, Cl, Br, CF₃, NO₂, CN, Methyl und Methoxy;
R(9) Wasserstoff;
X -CR(22)R(23)-;
R(22) und R(23)
Methyl,
sowie ihre physiologisch verträglichen Salze.

7. Verbindungen der Formel I gemäß einem oder mehreren der Ansprüche 1 bis 6 und ihre physiologisch verträglichen Salze zur Anwendung als Arzneimittel.

8. Pharmazeutische Zubereitung, enthaltend eine Menge von 0,1 bis 10 Gewichtsprozent mindestens einer Verbindung der Formel I nach einem oder mehreren der Ansprüche 1 bis 6 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

9. Verwendung einer Verbindung der Formel I nach einem oder mehreren der Ansprüche 1 bis 6 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

10. Verwendung einer Verbindung der Formel I nach einem oder mehreren der Ansprüche 1 bis 6 und/oder eines physiologisch verträglichen Salzes davon zur Herstellung eines Medikaments zum Inhibieren der Magensäuresekretion.

11. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Ulcera des Magens oder des intestinalen Bereiches.

12. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe der Refluxösophagitis.

13. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Durchfallerkrankungen.

14. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

15. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

16. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von atrialer Fibrillation oder atrialem Flattern.

17. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmem.

18. Verwendung einer Verbindung der Formel I nach einem oder mehreren der Ansprüche 1 bis 6 und/oder eines physiologisch verträglichen Salzes davon zur Herstellung eines Medikaments zur Therapie der Herzinsuffizienz.

19. Verwendung einer Verbindung der Formel I nach einem oder mehreren der Ansprüche 1 bis 2 und/oder eines physiologisch verträglichen Salzes davon zum Herstellen eines Medikaments zum Inhibieren der stimulierten Magensäuresekretion, zur Therapie oder Prophylaxe von Ulcera des Magens oder des intestinalen Bereiches, der Refluxösophagitis, von Durchfallerkrankungen, zur Therapie oder Prophylaxe von Arrhythmien, einschließlich atrialer, ventrikulärer und supraventrikulärer Arrhythmien, atrialer Fibrillation und atrialem Flattern und von Reentry-Arrhythmien, oder zur Verhinderung des plötzlichen Herztodes infolge von Kammerflimmern.

20. Pharmazeutische Zubereitung, enthaltend eine Menge von 0,1 bis 10 Gewichtsprozent mindestens einer Verbindung der Formel I nach einem oder mehreren der Ansprüche 1 bis 6 und/oder eines physiologisch verträglichen Salzes davon sowie eines Beta-blockers als Wirkstoffe, zusammen mit pharmazeutisch annehmbaren Träger- und Zusatzstoffen.

## Claims

1. A compound of the formula I 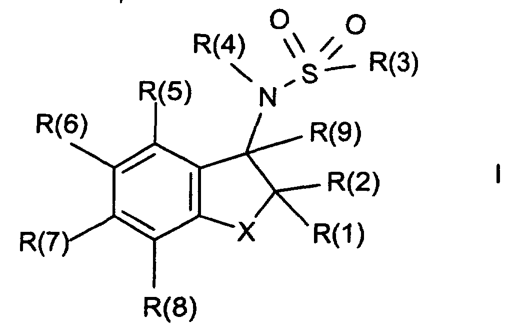 in which:
R(1) and R(2)
independently of one another are hydrogen, CF₃, alkyl having 1, 2, 3, 4, 5 or 6 carbon atoms;
or R(2)
is -OR(10a), if X is -CR(22)R(23)-;
R(10a) is hydrogen, acetyl or alkyl having 1, 2 or 3 carbon atoms;
R(3) is R(10b)-CₙH₂ₙ-NR(11)- or R(10b)-CₙH₂ₙ-,
where a CH₂ group in the groups CₙH₂ₙ may be replaced by -O-, -CO-, -S-, -SO-, -SO₂- or -NR(12a)-;
R(12a) is hydrogen, methyl or ethyl;
R(10b) is methyl, cycloalkyl having 3, 4, 5, 6, 7 or 8 carbon atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 carbon atoms;
or
R(10b) and R(11)
together are a bond if n is greater than 2;
or
R(3) together with R(4)
is an alkylene chain having 3, 4, 5, 6, 7 or 8 carbon atoms,
where a CH₂ group of the alkylene chain may be replaced by -O-, -CO-, -S-, -SO-, -SO₂- or -NR(12a)-; R(12a) is hydrogen, methyl or ethyl;
R(4) is R(13)-CᵣH₂ᵣ,
where a CH₂ group of the group CᵣH₂ᵣ may be replaced by -O-, -CH=CH-, -C≡C-, -CO-, -CO-O-, -O-CO-, -S-, -SO-, -SO₂-, -NR(14)- or -CONR(14)-;
R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl;
y is 2 or 3;
R(13) is CH₃, CF₃, C₂F₅, C₃F₇, cycloalkyl having 3, 4, 5, 6, 7 or 8 carbon atoms, -NR(15)R(16), -CONR(15)R(16), -OR(17), -COOR(17), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents, selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2, 3 or 4 carbon atoms;
or
R(15) and R(16)
together are a chain of 4 or 5 methylene groups of which one CH₂ group may be replaced by -O-, -S-, -NH-, -N(CH₃)- or -N(benzyl)-;
R(17) is hydrogen, alkyl having 1, 2 or 3 carbon atoms, -C_{z}H_{2z}OR(12c);
R(12c) is hydrogen, methyl or ethyl;
z is 2 or 3;
r is zero, 1, 2, 3, 4, 5, 6, 7, 8, 9, 10, 11, 12, 13, 14, 15, 16, 17, 18, 19 or 20;
R(5), R(6), R(7) and R(8)
independently of one another are hydrogen, F, Cl, Br, I, alkyl having 1, 2, 3, 4 or 5 carbon atoms, cycloalkyl having 3, 4, 5, 6, 7 or 8 carbon atoms, -CN, -CF₃, -C₂F₅, -C₃F₇, -N₃, -NO₂, -Y-CₛH₂ₛ-R(18), phenyl, thienyl, furyl or a nitrogen-containing heterocycle having 1, 2, 3, 4, 5, 6, 7, 8 or 9 carbon atoms,
where phenyl, thienyl, furyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -O-SO₂-, -SO₂NR(10c)- or -CONR(10c)-, where the link to the skeleton is in each case effected through the atom on the left; R(10c) is hydrogen or alkyl having 1, 2 or 3 carbon atoms;
s is zero, 1, 2, 3, 4, 5 or 6;
R(18) is hydrogen, methyl, CF₃, C₂F₅, C₃F₇, cycloalkyl having 3, 4, 5, 6, 7 or 8 carbon atoms, -OR(21), -COOR(21), -NR(15a)R(16a), -CONR(15a)R(16a), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15a) and R(16a)
independently of one another are hydrogen or alkyl having 1, 2, 3 or 4 carbon atoms;
or
R(15a) and R(16a)
together are a chain of 4 or 5 methylene groups of which one CH₂ group may be replaced by -O-, -S-, -NH-, -N(CH₃)- or -N(benzyl)-;
R(21) is hydrogen or alkyl having 1, 2 or 3 carbon atoms;
R(9) is hydrogen;
X is -CR(22)R(23)-, -O-, -NR(24)-, -S-, -SO-, -SO₂-;
R(22) and R(23)
independently of one another are hydrogen, CF₃, alkyl having 1, 2, 3, 4, 5 or 6 carbon atoms;
R(24) is hydrogen, alkyl having 1, 2, 3, 4, 5 or 6 carbon atoms or phenyl which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or its physiologically tolerable salts.

2. The compound of the formula I as claimed in claim 1 in which:
R(1) is hydrogen;
R(2) is hydrogen or -OR(10a);
R(10a) is hydrogen, acetyl or alkyl having 1, 2 or 3 carbon atoms;
R(3) is R(10b)-CₙH₂ₙ-NR(11)- or R(10b)-CₙH₂ₙ-,
where a CH₂ group in the groups CₙH₂ₙ may be replaced by -O-, -CO-, -S-, -SO-, -SO₂- or -NR(12a)-;
R(12a) is hydrogen, methyl or ethyl;
R(10b) is methyl, cycloalkyl having 3, 4, 5, 6, 7 or 8 carbon atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 carbon atoms;
or
R(10b) and R(11)
together are a bond if n is greater than 2;
or
R(3) together with R(4)
is an alkylene chain having 3, 4, 5, 6, 7 or 8 carbon atoms,
where a CH₂ group of the alkylene chain may be replaced by -O-, -CO-, -S-, -SO-, -SO₂- or -NR(12a)-; R(12a) is hydrogen, methyl or ethyl;
R(4) is R(13)-CᵣH₂ᵣ,
where a CH₂ group of the group CᵣH₂ᵣ may be replaced by -O-, -CH=CH-, -C≡C-, -CO-, -CO-O-, -O-CO-, -S-, -SO-, -SO₂-, -NR(14)- or -CONR(14)-;
R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl;
y is 2 or 3;
R(13) is CH₃, CF₃, C₂F₅, C₃F₇, cycloalkyl having 3, 4, 5, 6, 7 or 8 carbon atoms, -NR(15)R(16), -CONR(15)R(16), -OR(17), -COOR(17), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2, 3 or 4 carbon atoms;
or
R(15) and R(16)
together are a chain of 4 or 5 methylene groups of which one CH₂ group may be replaced by -O-, -S-, -NH-, -N(CH₃)- or -N(benzyl)-;
R(17) is hydrogen, alkyl having 1, 2 or 3 carbon atoms, -C_{z}H_{2z}OR(12c);
R(12c) is hydrogen, methyl or ethyl;
z is 2 or 3;
r is zero, 1, 2, 3, 4, 5, 6, 7, 8, 9, 10, 11, 12, 13, 14, 15, 16, 17, 18, 19 or 20;
R(5), R(6), R(7) and R(8)
independently of one another are hydrogen, F, Cl, Br, I, alkyl having 1, 2, 3, 4 or 5 carbon atoms, cycloalkyl having 3, 4, 5, 6, 7 or 8 carbon atoms, -CN, -CF₃, -C₂F₅, -C₃F₇, -N₃, -NO₂, -Y-CₛH₂ₛ-R(18), phenyl, thienyl, furyl or a nitrogen-containing heterocycle having 1, 2, 3, 4, 5, 6, 7, 8 or 9 carbon atoms,
where phenyl, thienyl, furyl and the nitrogen-containing heterocycle are unsubstituted or substituted by 1 or 2 substituents selected from the group consisting of F, Cl, Br, I, CF₃, NO2, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -O-SO₂-, -SO₂NR(10c) or -CONR(10c)-, where the link to the skeleton is in each case effected through the atom on the left;
R(10c) is hydrogen or alkyl having 1, 2 or 3 carbon atoms;
s is zero, 1, 2, 3, 4, 5 or 6;
R(18) is hydrogen, methyl, CF₃, C₂F₅, C₃F₇, cycloalkyl having 3, 4, 5, 6, 7 or 8 carbon atoms, -OR(21), -COOR(21),
-NR(15a)R(16a), -CONR(15a)R(16a), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15a) and R(16a)
independently of one another are hydrogen or alkyl having 1, 2, 3 or 4 carbon atoms;
or
R(15a) and R(16a)
together are a chain of 4 or 5 methylene groups of which one CH₂ group may be replaced by -O-, -S-, -NH-, -N(CH₃)- or -N(benzyl)-;
R(21) is hydrogen or alkyl having 1, 2 or 3 carbon atoms;
R(9) is hydrogen;
X is -CR(22)R(23)-;
R(22) and R(23)
independently of one another are hydrogen, CF₃, alkyl having 1, 2, 3, 4, 5 or 6 carbon atoms,
or its physiologically tolerable salts.

3. The compound of the formula I as claimed in claims 1 or 2 in which:
R(1) is hydrogen;
R(2) is hydrogen or -OR(10a);
R(10a) is hydrogen, acetyl or alkyl having 1, 2 or 3 carbon atoms;
R(3) is R(10b)-CₙH₂ₙ-,
R(10b) is methyl, cycloalkyl having 3, 4, 5 or 6 carbon atoms, CF₃, C₂F₅ or C₃F₇;
n is zero, 1, 2, 3, 4 or 5;
R(4) is R(13)-CᵣH₂ᵣ,
where a CH₂ group of the group CᵣH₂ᵣ may be replaced by -O-, -CO-, -CO-O-, -O-CO-, -NR(14)- or -CONR(14)-; R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl;
y is 2 or 3;
R(13) is CH₃, CF₃, C₂F₅, C₃F₇, cycloalkyl having 3, 4, 5 or 6 carbon atoms, -NR(15)R(16), -CONR(15)R(16), -OR(17), -COOR(17), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2, 3 or 4 carbon atoms;
or
R(15) and R(16)
together are a chain of 4 or 5 methylene groups of which one CH₂ group may be replaced by -O-, -S-, -NH-, -N(CH₃)- or -N(benzyl)-;
R(17) is hydrogen or alkyl having 1, 2 or 3 carbon atoms;
r is zero, 1, 2, 3, 4, 5, 6, 7, 8, 9 or 10;
R(5), R(6), R(7) and R(8)
independently of one another are hydrogen, F, Cl, Br, alkyl having 1, 2, 3, 4 or 5 carbon atoms, cycloalkyl having 3, 4, 5 or 6 carbon atoms, -CN, -CF₃, -C₂F₅, -C₃F₇, -NO₂, -Y-CₛH₂ₛ-R(18), phenyl, thienyl, furyl or a nitrogen-containing heterocycle having 1, 2, 3, 4, 5, 6, 7, 8 or 9 carbon atoms;
where phenyl, thienyl, furyl and the nitrogen-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-;
s is zero, 1, 2, 3, 4, 5 or 6;
R(18) is hydrogen, methyl, CF₃, C₂F₅, C₃F₇, cycloalkyl having 3, 4, 5 or 6 carbon atoms, -OR(21), -COOR(21), -NR(15a)R(16a), -CONR(15a)R(16a), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
R(15a) and R(16a)
independently of one another are hydrogen or alkyl having 1, 2, 3 or 4 carbon atoms;
or
R(15a) and R(16a)
together are a chain of 4 or 5 methylene groups of which one CH₂ group may be replaced by -O-, -S-, -NH-, -N(CH₃)- or -N(benzyl)-;
R(21) is alkyl having 1, 2 or 3 carbon atoms;
R(9) is hydrogen;
X is -CR(22)R(23)-;
R(22) and R(23)
independently of one another are hydrogen, CF₃, alkyl having 1, 2 or 3 carbon atoms,
or its physiologically tolerable salts.

4. The compound of the formula I as claimed in one or more of claims 1 to 3, in which:
R(1) is hydrogen;
R(2) is hydrogen or -OR(10a);
R(10a) is hydrogen or methyl;
R(3) is R(10b)-CₙH₂ₙ-,
R(10b) is methyl;
n is zero, 1 or 2;
R(4) is R(13)-CᵣH₂ᵣ,
where a CH₂ group of the group CᵣH₂ᵣ may be replaced by -O-, -CO-O-, or -CONR(14)-;
R(14) is hydrogen, alkyl having 1, 2 or 3 carbon atoms, -C_{y}H_{2y}-OR(12b);
R(12b) is hydrogen, methyl or ethyl;
y is 2 or 3;
R(13) is CH₃, CF₃ or a nitrogen-containing heterocycle having 1, 2, 3, 4, 5, 6, 7, 8 or 9 carbon atoms,
where the nitrogen-containing heterocycle is unsubstituted or substituted by 1 or 2 substituents selected from the group consisting of F, Cl, Br, CF₃, NO₂, CN, methyl or methoxy;
r is zero, 1, 2, 3, 4, 5, 6, 7 or 8;
R(5), R(7) and R(8)
are hydrogen;
R(6) is hydrogen, F, Cl, Br, alkyl having 1, 2, 3, 4 or 5 carbon atoms, cycloalkyl having 3, 4, 5 or 6 carbon atoms, -CN, -CF₃, -C₂F₅, -C₃F₇, -NO₂, -Y-CₛH₂ₛ-R(18), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, CF₃, NO₂, CN, methyl and methoxy;
Y is -O-;
s is 1, 2, 3, 4, 5 or 6;
R(18) is hydrogen, CF₃, -OR(21), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, CF₃, NO₂, CN, methyl and methoxy;
R(21) is alkyl having 1, 2 or 3 carbon atoms;
R(9) is hydrogen;
X is -CR(22)R(23)-;
R(22) and R(23)
independently of one another are CF₃ or methyl,
or its physiologically tolerable salts.

5. The compound of the formula I as claimed in one or more of claims 1 to 4, in which:
R(1) is hydrogen;
R(2) is hydrogen;
R(3) is methyl or ethyl;
R(4) is R(13)-CᵣH₂ᵣ,
where a CH₂ group of the group CᵣH₂ᵣ may be replaced by -O-;
R(13) is CH₃ or CF₃;
r is zero, 1, 2, 3, 4, 5 or 6;
R(5), R(7) and R(8)
are hydrogen;
R(6) is F, Cl, Br, alkyl having 1, 2, 3, 4 or 5 carbon atoms, -CN, -CF₃, -NO₂, -Y-CₛH₂ₛ-R(18) or phenyl
which is unsubstituted or substituted by 1 or 2 substituents selected from the group consisting of F, Cl, Br, CF₃, NO₂, CN, methyl and methoxy;
Y is -O-;
s is 1, 2, 3, 4, 5 or 6;
R(18) is hydrogen, CF₃ or phenyl,
which is unsubstituted or substituted by 1 or 2 substituents selected from the group consisting of F, Cl, Br, CF₃, NO₂, CN, methyl and methoxy;
R(9) is hydrogen;
X is -CR(22)R(23)-;
R(22) and R(23)
are methyl,
or its physiologically tolerable salts.

6. The compound of the formula I as claimed in one or more of claims 1 to 4, in which:
R(1) is hydrogen;
R(2) is OH;
R(3) is methyl or ethyl;
R(4) is R(13)-CᵣH₂ᵣ,
where a CH₂ group of the group CᵣH₂ᵣ may be replaced by -O-;
R(13) is CH₃ or CF₃;
r is zero, 1, 2, 3, 4, 5 or 6;
R(5), R(7) and R(8)
are hydrogen;
R(6) is F, Cl, Br, alkyl having 1, 2, 3, 4 or 5 carbon atoms, -CN, -CF₃, -NO₂, -Y-CₛH₂ₛ-R(18) or phenyl
which is unsubstituted or substituted by 1 or 2 substituents selected from the group consisting of F, Cl, Br, CF₃, NO₂, CN, methyl and methoxy;
Y is -O-;
s is 1, 2, 3, 4, 5 or 6;
R(18) is hydrogen, CF₃ or phenyl,
which is unsubstituted or substituted by 1 or 2 substituents selected from the group consisting of F, Cl, Br, CF₃, NO₂, CN, methyl and methoxy;
R(9) is hydrogen;
X is -CR(22)R(23)-;
R(22) and R(23)
are methyl,
or its physiologically tolerable salts.

7. The compound of the formula I as claimed in one or more of claims 1 to 6 or its physiologically tolerable salts for use as a pharmaceutical.

8. A pharmaceutical preparation comprising an amount of from 0.1 to 10% by weight of at least one compound of the formula I as claimed in one or more of claims 1 to 6 and/or of a physiologically tolerable salt thereof as active compound, together with pharmaceutically acceptable excipients and additives and, if appropriate, additionally one or more other pharmacologically active compounds.

9. The use of a compound of the formula I as claimed in one or more of claims 1 to 6 and/or of a physiologically tolerable salt thereof for the production of a medicament having K⁺ channel-blocking action for the therapy and prophylaxis of K⁺ channel-mediated diseases.

10. The use of a compound of the formula I as claimed in one or more of claims 1 to 6 and/or of a physiologically tolerable salt thereof for the production of a medicament for the inhibition of gastric acid secretion.

11. The use of a compound of the formula I as claimed in one or more of claims 1 to 6 and/or of a physiologically tolerable salt thereof for the production of a medicament for the therapy or prophylaxis of ulcers of the stomach or of the intestinal region.

12. The use of a compound of the formula I as claimed in one or more of claims 1 to 6 and/or of a physiologically tolerable salt thereof for the production of a medicament for the therapy or prophylaxis of reflux esophagitis.

13. The use of a compound of the formula I as claimed in one or more of claims 1 to 6 and/or of a physiologically tolerable salt thereof for the production of a medicament for the therapy or prophylaxis of diarrheal illnesses.

14. The use of a compound of the formula I as claimed in one or more of claims 1 to 6 and/or of a physiologically tolerable salt thereof for the production of a medicament for the therapy or prophylaxis of all types of arrhythmias, including atrial, ventricular and supraventricular arrhythmias.

15. The use of a compound of the formula I as claimed in one or more of claims 1 to 6 and/or of a physiologically tolerable salt thereof for the production of a medicament for the therapy or prophylaxis of cardiac arrhythmias which can be eliminated by action potential prolongation.

16. The use of a compound of the formula I as claimed in one or more of claims 1 to 6 and/or of a physiologically tolerable salt thereof for the preparation of a medicament for the therapy or prophylaxis of atrial fibrillation or atrial flutters.

17. The use of a compound of the formula I as claimed in one or more of claims 1 to 6 and/or of a physiologically tolerable salt thereof for the production of a medicament for the therapy or prophylaxis of reentry arrhythmias or for preventing sudden heart death as a result of ventricular fibrillation.

18. The use of a compound of the formula I as claimed in one or more of claims 1 to 6 and/or of a physiologically tolerable salt thereof for the production of a medicament for the therapy of cardiac insufficiency.

19. The use of a compound of the formula I as claimed in one or more of claims 1 to 2 and/or of a physiologically tolerable salt thereof for the preparation of a medicament for the inhibition of stimulated gastric acid secretion, for the therapy or prophylaxis of ulcers of the stomach or of the intestinal region, of reflux esophagitis, of diarrheal diseases, for the therapy or prophylaxis of arrhythmias, including atrial, ventricular and supraventricular arrhythmias, atrial fibrillation and atrial flutters and of reentry arrhythmias, or for the prevention of sudden heart death as a result of ventricular fibrillation.

20. A pharmaceutical preparation comprising an amount of from 0.1 to 10% by weight of at least one compound of the formula I as claimed in one or more of claims 1 to 6 and/or of a physiologically tolerable salt thereof and of a beta blocker as active compounds, together with pharmaceutically acceptable excipients and additives.

## Revendications

1. Composés de formule I 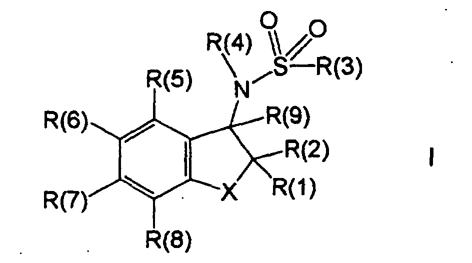 dans laquelle
R(1), R(2)
indépendamment l'un de l'autre sont l'hydrogène, un groupe CF₃, alkyle avec 1, 2, 3, 4, 5 ou 6 atomes de C ;
ou R(2)
est -OR(10a), quand X signifie -CR(22)R(23)- ;
R(10a) est l'hydrogène, un groupe acétyle ou alkyle avec 1, 2 ou 3 atomes de C ;
R(3)
est R(10b)-CₙH₂ₙ-NR(11)- ou R(10b)-CₙH₂ₙ-,
où un groupe CH₂ dans les groupes CₙH₂ₙ peut être remplacé par -O-, -CO-, -S-, -SO-, -SO₂- ou -NR(12a)-;
R(12a) est l'hydrogène, un groupe méthyle ou éthyle ;
R(10b) est un groupe méthyle, cycloalkyle avec 3, 4, 5, 6, 7 ou 8 atomes de C, un groupe CF₃, C₂F₅ ou C₃F₇ ;
n vaut 0, 1, 2, 3, 4, 5, 6, 7, 8, 9 ou 10 ;
R(11) est l'hydrogène, un groupe alkyle avec 1, 2, 3, 4, 5 ou 6 atomes de C ;
ou
R(10b) et R(11)
forment ensemble une liaison, pour autant que n est supérieur à 2;
ou
R(3) avec R(4) forment ensemble
une chaîne alkylène avec 3, 4, 5, 6, 7 ou 8 atomes de C,
où un groupe CH₂ de la chaîne alkyle peut être remplacé par -O-, -CO-, -S-, -SO-, -SO₂- ou -NR(12a)- ;
R(12a) est l'hydrogène, un groupe méthyle ou éthyle ;
R(4) est R(13)-CᵣH₂ᵣ,
où un groupe CH₂ du groupe CᵣH₂ᵣ peut être remplacé par -O-, -CH=CH-, -C=C-, -CO-, -CO-O-, -O-CO-, -S-, -SO₂-, -NR(14)- ou -CONR(14)- ;
R(14) est l'hydrogène, un groupe alkyle avec 1, 2 ou 3 atomes de C, -C_{y}H_{2y}-OR(12b), -C_{y}H_{2y}-NR(12b)₂;
R(12b) est l'hydrogène, un groupe méthyle ou éthyle ;
y vaut 2 ou 3 ;
R(13) est un groupe CH₃, CF₃, C₂F₅, C₃F₇, cycloalkyle avec 3, 4, 5, 6, 7 ou 8 atomes de C, -NR(15)R(16), -CONR(15)R(16), -OR(17), -COOR(17), phényle ou un hétérocycle contenant N avec 1, 2, 3, 4, 5, 6, 7, 8 ou 9 atomes de C,
où le groupe phényle et l'hétérocycle contenant N sont non substitués ou substitués par 1 ou 2 substituants, choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino;
R(15) et R(16)
indépendamment l'un de l'autre sont l'hydrogène ou un groupe alkyle avec 1, 2, 3 ou 4 atomes de C ;
ou
R(15) et R(16)
forment ensemble un chaîne de 4 ou 5 groupes méthylène, dont un groupe CH₂ peut être remplacé par -O-, -S-, -NH-, -N(CH₃)- ou -N(benzyl)-
R(17) est l'hydrogène, un groupe alkyle avec 1, 2 ou 3 atomes de C, C_{z}H_{2z}OR(12c);
R(12c) est l'hydrogène, un groupe méthyle ou éthyle ;
z vaut 2 ou 3 ;
r vaut 0, 1, 2, 3, 4, 5, 6, 7, 8, 9, 10, 11, 12, 13, 14, 15, 16, 17, 18, 19 ou 20 ;
R(5), R(6), R(7) et R(8)
indépendamment l'un de l'autre sont l'hydrogène, F, Cl, Br, I, un groupe alkyle avec 1, 2, 3, 4 ou 5 atomes de C, cycloalkyle avec 3, 4, 5, 6, 7 ou 8 atomes de C, -CN, -CF₃-, -C₂F₅, -C₃F₇, -N₃, -NO₂, -Y-CₛH₂ₛ-R(18), phényle, thiényle, furyle ou un hétérocycle contenant N avec 1, 2, 3, 4, 5, 6, 7, 8 ou 9 atomes de C,
où les groupes phényle, thiényle, furyle et l'hétérocycle contenant N sont non substitués ou substitués par 1 ou 2 substituants choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino ;
Y est -O-, -CO-, -CO-O-, -O-CO-, -S-, -SO-, -SO₂-, -O-SO₂-, -SO₂NR(10c)- ou -CONR(10c)-, où la liaison avec le squelette de base se fait à chaque fois sur l'atome se trouvant à gauche ;
R(10c) est l'hydrogène ou un groupe alkyle avec 1, 2 ou 3 atomes de C ;
s vaut 0, 1, 2, 3, 4, 5 ou 6 ;
R(18) est l'hydrogène, un groupe méthyle, CF₃, -C₂F₅, -C₃F₇, cycloalkyle avec 3, 4, 5, 6, 7 ou 8 atomes de C, -OR(21), -COOR(21), -NR(15a)R(16a), -CONR(15a)R(16a), phényle ou un hétérocycle contenant N avec 1, 2, 3, 4, 5, 6, 7, 8 ou 9 atomes de C,
où le groupe phényle et l'hétérocycle contenant N sont non substitués ou substitués par 1 ou 2 substituants choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino ;
R(15a) et R(16a)
indépendamment l'un de l'autre sont l'hydrogène ou un groupe alkyle avec 1, 2, 3 ou 4 atomes de C ;
ou
R(15a) et R(16a)
forment ensemble une chaîne de 4 ou 5 groupes méthyle, dont un groupe -CH₂- peut être remplacé par -O-, -S-, -NH-, -N(CH₃)-ou -N(benzyl)- ;
R(21) est l'hydrogène, un groupe alkyle avec 1, 2 ou 3 atomes de C;
R(9) est l'hydrogène ;
X est -CR(22)R(23)-, -O-, -NR(24)-, -S-, -SO-, -SO₂- ;
R(22) et R(23) indépendamment l'un de l'autre sont l'hydrogène, un groupe CF₃, alkyle avec 1, 2, 3, 4, 5 ou 6 atomes de C ;
R(24) est l'hydrogène, un groupe alkyle avec 1, 2, 3, 4, 5 ou 6 atomes de C ou phényle,qui est non substitué ou substitué par 1 ou 2 substituants choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino ;
ainsi que leurs sels compatibles du point de vue physiologique.

2. Composés de formule I selon la revendication 1, dans laquelle:
R(1) est l'hydrogène;
R(2) est l'hydrogène ou -OR(10a);
R(10a) est l'hydrogène, un groupe acétyle ou alkyle avec 1, 2 ou 3 atomes de C ;
R(3) est R(10b)-CₙH₂ₙ-NR(11)-ou R(10b)-CₙH₂ₙ-,
où un groupe CH₂ dans les groupes CₙH₂ₙ peut être remplacé par -O-, -CO-, -S-, -SO-, -SO₂- ou -NR(12a)-; R(12a) est l'hydrogène, un groupe méthyle ou éthyle ;
R(10b) est un groupe méthyle, cycloalkyle avec 3, 4, 5, 6, 7 ou 8 atomes de C, CF₃, C₂F₅ ou C₃F₇ ;
n vaut 0, 1, 2, 3, 4, 5, 6, 7, 8, 9 ou 10 ;
R(11) est l'hydrogène ou un groupe alkyle avec 1, 2, 3, 4, 5 ou 6 atomes de C ;
ou
R(10b) et R(11)
forment ensemble une liaison, pour autant que n soit supérieur à 2;
ou
R(3) forment ensemble avec R(4)
une chaîne alkylène avec 3, 4, 5, 6, 7 ou 8 atomes de C,
où un groupe CH₂ de la chaîne alkylène peut être rempalcé par -O-, -CO-, -S-, -SO-, -SO₂- ou -NR(12a)-;
R(12a) est l'hydrogène, un groupe méthyle ou éthyle ;
R(4) est R(13)-CᵣH₂ᵣ,
où un groupe CH₂ de la chaîne CᵣH₂ᵣ peut être remplacé par -O-, -CH=CH-, -C=C-, -CO-, -CO-O, -O-CO-, -S-, -SO-, -SO₂-, -NR(14)- ou -CONR(14)- ;
R(14) est l'hydrogène, un groupe alkyle avec 1, 2 ou 3 atomes de C, -C_{y}H_{2y}-OR(12b), -C_{y}H_{2y}-NR(12b)₂ ;
R(12b) est l'hydrogène, un groupe méthyle ou éthyle ;
y vaut 2 ou 3 ;
R(13) est un groupe CH₃, CF₃, C₂F₅, C₃F₇, cycloalkyle avec 3, 4, 5, 6, 7 ou 8 atomes de C, -NR(15)R(16), -CONR(15)R(16), -OR(17), -COOR(17), phényle ou un hétérocycle contenant N avec 1, 2, 3, 4, 5, 6, 7, 8 ou 9 atomes de C,
où le groupe phényle et hétérocycle contenant N sont non substitués ou substitués par 1 ou 2 susbtituants choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino ;
R(15) et R(16)
indépendamment l'un de l'autre sont l'hydrogène ou un groupe alkyle avec 1, 2, 3 ou 4 atomes de C ;
ou
R(15) et R(16)
ensemble forment une chaîne de 4 ou 5 groupes méthylène dont un groupe CH₂ peut être remplacé par -O-, -S-, -NH-, -N(CH₃)-ou -N(benzyl)-,
R(17) est l'hydrogène, un groupe alkyle avec 1, 2 ou 3 atomes de C, C_{z}H_{2z}OR(12c);
R(12c) est l'hydrogène, un groupe méthyle ou éthyle ;
z vaut 2 ou 3 ;
r vaut 0, 1, 2, 3, 4, 5, 6, 7, 8, 9, 10, 11, 12, 13, 14, 15, 16, 17, 18, 19 ou 20;
R(5), R(6), R(7) et R(8)
indépendamment l'un de l'autre sont l'hydrogène, F, Cl, Br, I, un groupe alkyle avec 1, 2, 3, 4 ou 5 atomes de C, cycloalkyle avec 3, 4, 5, 6, 7 ou 8 atomes de C, -CN, -CF₃, -C₂F₅, -C₃F₇, -N₃, -NO₂, -Y-CₛH₂ₛ-R(18), phényle, thiényle, furyle ou un hétérocycle contenant N avec 1, 2, 3, 4, 5, 6, 7, 8 ou 9 atomes de C,
où les groupes phényle, thiényle, furyle et l'hétérocycle contenant N sont non substitués ou substitués par 1 ou 2 substituants, choisis dans le groupe constitué par F, Cl, Br, I, CF₃, NO₂, CN, NH₂, OH, un groupe méthyle, éthyle, méthoxy, diméthylamino, sulfamoyle, méthylsulfonyle et méthylsulfonylamino ;
Y est -O-, -CO-, -CO-O-, -C-CO-, -S-, -SO-, -SO₂-, -SO₂NR(10c) ou -CONR(10c)-, où la liaison avec le squelette de base se fait à chaque fois sur l'atome se trouvant à gauche ;
R(10c) est l'hydrogène ou un groupe alkyle avec 1, 2 ou 3 atomes de C ;
s vaut 0, 1 ,2 ,3 ,4 ,5 ou 6 ;
R(18) est l'hydrogène, un groupe méthyle, CF₃, C₂F₅, C₃F₇, cycloalkyle avec 3, 4, 5, 6, 7 ou 8 atomes de C, -OR(21), -COOR(21), -NR(15a)R(16a), -CONR(15a)R(16a), phényle ou un hétérocycle contenant N avec 1, 2, 3, 4, 5, 6, 7, 8 ou 9 atomes de C,
où le groupe phényle et l'hétérocycle contenant N sont non substitués ou substitués avec 1 ou 2 substituants, choisis dans le groupe constitué par F, Cl, Br, I, CF₃, NO₂, CN, NH₂, OH, un groupe méthyle, éthyle, méthoxy, diméthylamino, sulfamoyle, méthylsulfonyle et méthylsulfonylamino ;
R(15) et R(16a)
indépendamment l'un de l'autre sont l'hydrogène ou un groupe alkyle avec 1, 2, 3 ou 4 atomes de C ;
ou
R(15a) et R(16a)
forment ensemble une chaîne de 4 ou 5 groupes méthylène, dont un groupe CH₂ peut être remplacé par -O-, -S-, -NH-, -N(CH₃)- ou - N(benzyl) ;
R(21) est l'hydrogène ou un groupe alkyle avec 1, 2 ou 3 atomes de C;
R(9) est l'hydrogène ;
X est -CR(22)R(23)- ;
R(22) et R(23) indépendamment l'un de l'autre sont l'hydrogène, CF₃, un groupe alkyle avec 1, 2, 3, 4, 5 ou 6 atomes de C ;
ainsi que leurs sels compatibles du point de vue physiologique.

3. Composés de formule I selon les revendications 1 ou 2, dans laquelle :
R(1) est l'hydrogène;
R(2) est l'hydrogène ou -OR(10a) ;
R(10a) est l'hydrogène, un groupe acétyle ou alkyle avec 1, 2 ou 3 atomes de C ;
R(3) est R(10b)-CₙH₂ₙ-,
R(10b) est un groupe méthyle, cycloalkyle avec 3, 4, 5 ou 6 atomes de C, CF₃, C₂F₅ ou C₃F₇ ;
n vaut 0, 1, 2, 3, 4 ou 5 ;
R(4) est R(13)-CᵣH₂ᵣ,
où un groupe CH₂ du groupe CᵣH₂ᵣ peut être remplacé par -O-, -CO-, -CO-O-, -O-CO-, -NR(14)- ou -CONR(14)- ; R(14) est l'hydrogène, un groupe alkyle avec 1, 2 ou 3 atomes de C, -C_{y}H_{2y}-OR(12b), -C_{y}H_{2y}-NR(12b)₂ ;
R(12b) est l'hydrogène, un groupe méthyle ou éthyle ;
y vaut 2 ou 3 ;
R(13) est un groupe CH₃, CF₃, C₂F₅, C₃F₇, cycloalkyle avec 3, 4, 5 ou 6 atomes de C, -NR(15)R(16), -CONR(15)R(16), -OR(17), -COOR(17), phényle ou un hétérocycle contenant N avec 1, 2, 3, 4, 5, 6, 7, 8 ou 9 atomes de C,
où le groupe phényle et l'hétérocycle contenant N sont non substitués ou substitués par 1 ou 2 susbtituants choisis dans le groupe constitué par F, Cl, Br, CF₃, NO₂, CN, NH₂, OH, méthyle, éthyle, méthoxy, diméthylamino, sulfamoyle, méthylsulfonyle et méthylsulfonylamino ;
R(15) et R(16)
indépendamment l'un de l'autre sont l'hydrogène ou un groupe alkyle avec 1, 2, 3 ou 4 atomes de C ;
ou
R(15) et R(16)
ensemble forment une chaîne de 4 ou 5 groupes méthylène dont un groupe CH₂ peut être remplacé par -O-, -S-, -NH-, -N(CH₃)- ou -N(benzyl)-,
R(17) est l'hydrogène, un groupe alkyle avec 1, 2 ou 3 atomes de C;
r vaut 0, 1, 2, 3, 4, 5, 6, 7, 8, 9 ou 10 ;
R(5), R(6), R(7) et R(8)
indépendamment l'un de l'autre sont l'hydrogène, F, Cl, Br, un groupe alkyle avec 1, 2, 3, 4 ou 5 atomes de C, cycloalkyle avec 3, 4, 5 ou 6 atomes de C, -CN, -CF₃, -C₂F₅, -C₃F₇, -NO₂, -Y-CₛH₂ₛ-R(18), phényle, thiényle, furyle ou un hétérocycle contenant N avec 1, 2, 3, 4, 5, 6, 7, 8 ou 9 atomes de C,
où les groupes phényle, thiényle, furyle et l'hétérocycle contenant N sont non substitués ou substitués par 1 ou 2 substituants, choisis dans le groupe constitué par F, Cl, Br, CF₃, NO₂, CN, NH₂, OH, un groupe méthyle, éthyle, méthoxy, diméthylamino, sulfamoyle, méthylsulfonyle et méthylsulfonylamino ;
Y est -O-, -CO-, -CO-O-, -O-CO-, -S-, -SO-, -SO₂-;
s vaut 0, 1 ,2 ,3 ,4 ,5 ou 6 ;
R(18) est l'hydrogène, un groupe méthyle, CF₃, C₂F₅, C₃F₇, cycloalkyle avec 3, 4, 5 ou 6 atomes de carbone, -OR(21), -COOR(21), -NR(15a)R(16a), -CONR(15a)R(16a), phényle ou un hétérocycle contenant N avec 1, 2, 3, 4, 5, 6, 7, 8 ou 9 atomes de C,
où le groupe phényle et l'hétérocycle contenant N sont non substitués ou substitués avec 1 ou 2 substituants, choisis dans le groupe constitué par F, Cl, Br, CF₃, NO₂, CN, NH₂, OH, un groupe méthyle, éthyle, méthoxy, diméthylamino, sulfamoyle, méthylsulfonyle et méthylsutfonylamino ;
R(15) et R(16a)
sont indépendamment l'un de l'autre l'hydrogène ou un groupe alkyle avec 1, 2, 3 ou 4 atomes de C ;
ou
R(15a) et R(16a)
forment ensemble une chaîne de 4 ou 5 groupes méthylène, dont un groupe CH₂ peut être remplacé par -O-, -S-, -NH-, -N(CH₃)- ou - N(benzyl) ;
R(21) est un groupe alkyle avec 1, 2 ou 3 atomes de C ;
R(9) est l'hydrogène ;
X est -CR(22)R(23)- ;
R(22) et R(23)
indépendamment l'un de l'autre sont l'hydrogène, CF₃, un groupe alkyle avec 1, 2 ou 3 atomes de C ;
ainsi que leurs sels compatibles du point de vue physiologique.

4. Composés de formule I selon une ou plusieurs des revendications 1 à 3, dans laquelle :
R(1) est l'hydrogène;
R(2) est l'hydrogène ou -OR(10a) ;
R(10a) est l'hydrogène ou un groupe méthyle ;
R(3) est R(10b)-CₙH₂ₙ-,
R(10b) est un groupe méthyle ;
n vaut 0, 1 ou 2 ;
R(4) est R(13)-CᵣH₂ᵣ,
où un groupe CH₂ de la chaîne CᵣH₂ᵣ peut être remplacé par -O-, -CO-O-, ou -CONR(14)-;
R(14) est l'hydrogène, un groupe alkyle avec 1, 2 ou 3 atomes de C, -C_{y}H_{2y}-OR(12b) ;
R(12b) est l'hydrogène, un groupe méthyle ou éthyle ;
y vaut 2 ou 3 ;
R(13) est un groupe CH₃, CF₃ ou un hétérocycle contenant N avec 1, 2, 3, 4, 5, 6, 7, 8 ou 9 atomes de C,
où le groupe phényle et l'hétérocycle contenant N sont non substitués ou substitués par 1 ou 2 susbtituants choisis dans le groupe constitué par F, Cl, Br, CF₃, NO₂, CN, méthyle ou méthoxy ;
r vaut 0, 1, 2, 3, 4, 5, 6, 7 ou 8 ;
R(5), R(7) et R(8)
sont l'hydrogène ;
R(6) est l'hydrogène, F, Cl, Br, un groupe alkyle avec 1, 2, 3, 4 ou 5 atomes de C, cycloalkyle avec 3, 4, 5 ou 6 atomes de C, -CN, -CF₃, -C₂F₅, -C₃F₇, -NO₂, -Y-CₛH₂ₛ-R(18), phényle ou un hétérocycle contenant N avec 1, 2, 3, 4, 5, 6, 7, 8 ou 9 atomes de C,
où les groupes phényle et l'hétérocycle contenant N sont non substitués ou substitués par 1 ou 2 substituants, choisis dans le groupe constitué par F, Cl, Br, CF₃, NO₂, CN, un groupe méthyle et méthoxy ;
Y est -O-;
s vaut 0, 1 ,2 ,3 ,4 ,5 ou 6 ;
R(18) est l'hydrogène, CF₃, -OR(21), phényle ou un hétérocycle contenant N avec 1, 2, 3, 4, 5, 6, 7, 8 ou 9 atomes de C,
où le groupe phényle et l'hétérocycle contenant N sont non substitués ou substitués avec 1 ou 2 substituants, choisis dans le groupe constitué par F, Cl, Br, CF₃, NO₂, CN, un groupe méthyle et méthoxy
R(21) est un groupe alkyle avec 1, 2 ou 3 atomes de C ;
R(9) est l'hydrogène ;
X est -CR(22)R(23)- ;
R(22) et R(23)
indépendamment l'un de l'autre sont CF₃, un groupe méthyle;
ainsi que leurs sels compatibles du point de vue physiologique.

5. Composés de formule I selon une ou plusieurs des revendications 1 à 4, dans laquelle :
R(1 ) est l'hydrogène ;
R(2) est l'hydrogène ;
R(3) est un groupe méthyle ou éthyle ;
R(4) est R(13)-CᵣH₂ᵣ,
où un groupe CH₂ du groupe CᵣH₂ᵣ peut être remplacé par -O-;
R(13) est un groupe CH₃ ou CF₃;
r vaut 0, 1, 2, 3, 4, 5 ou 6;
R(5), R(7) et R(8)
sont l'hydrogène ;
R(6) est F, Cl, Br, un groupe alkyle avec 1, 2, 3, 4 ou 5 atomes de C, -CN, -CF₃, -NO₂, -Y-CₛH₂ₛ-R(18) ou phényle
qui est non substitué ou substitué par 1 ou 2 substituants, choisis dans le groupe constitué par F, Cl, Br, CF₃, NO₂, CN, un groupe méthyle et méthoxy ;
Y est -O- ;
s vaut 0, 1 ,2 ,3 ,4 ,5 ou 6 ;
R(18) est l'hydrogène, CF₃ ou un groupe phényle,
qui est non substitué ou substitué avec 1 ou 2 substituants, choisis dans le groupe constitué par F, Cl, Br, CF₃, NO₂, CN, un groupe méthyle et méthoxy ;
R(9) est l'hydrogène ;
X est -CR(22)R(23)- ;
R(22) et R(23)
sont un groupe méthyle ;
ainsi que leurs sels compatibles du point de vue physiologique.

6. Composés de formule I selon une ou plusieurs des revendications 1 à 4, dans laquelle :
R(1) est l'hydrogène ;
R(2) est OH ;
R(3) est un groupe méthyle ou éthyle ;
R(4) est R(13)-CᵣH₂ᵣ,
où un groupe CH₂ du groupe CᵣH₂ᵣ peut être remplacé par -O-;
R(13) est un groupe CH₃ ou CF₃;
r vaut 0, 1, 2, 3, 4, 5 ou 6 ;
R(5), R(7) et R(8)
sont l'hydrogène ;
R(6) est F, Cl, Br, un groupe alkyle avec 1, 2, 3, 4 ou 5 atomes de C, -CN, -CF₃, -NO₂, -Y-CₛH₂ₛ-R(18) ou phényle
qui est non substitué ou substitué par 1 ou 2 substituants, choisis dans le groupe constitué par F, Cl, Br, CF₃, NO₂, CN, un groupe méthyle et méthoxy ;
Y est -O- ;
s vaut 0, 1 ,2 ,3 ,4 ,5 ou 6 ;
R(18) est l'hydrogène, CF₃ ou un groupe phényle,
qui est non substitué ou substitué avec 1 ou 2 substituants, choisis dans le groupe constitué par F, Cl, Br, CF₃, NO₂, CN, un groupe méthyle et méthoxy ;
R(9) est l'hydrogène ;
X est -CR(22)R(23)- ;
R(22) et R(23)
sont un groupe méthyle ;
ainsi que leurs sels compatibles du point de vue physiologique.

7. Composés de formule I selon l'une ou plusieurs des revendications 1 à 6 et leurs sels compatibles du point de vue physiologique pour l'utilisation comme médicament.

8. Préparation pharmaceutique contenant une quantité de 0,1 à 10% en poids d'au moins un composé de formule I selon une ou plusieurs des revendications 1 à 6 et/ou un sel compatible du point de vue physiologique de celui-ci comme composé actif, avec des véhicules ou des additifs acceptables du point de vue pharmaceutique et éventuellement encore un ou plusieurs autres composés actifs du point de vue pharmacologique.

9. Utilisation d'un composé de formule I selon une ou plusieurs des revendications 1 à 6 et/ou un sel compatible du point de vue physiologique de celui-ci pour la préparation d'un médicament avec une action bloquant le canal K⁺ pour la thérapie et la prophylaxie de maladies engendrées par le canal K⁺.

10. Utilisation du composé de formule I selon une ou plusieurs des revendications 1 à 6 et/ou d'un sel compatible du point de vue physiologique de celui-ci pour la préparation d'un médicament pour inhiber la sécrétion de l'acide gastrique.

11. Utilisation du composé de formule I selon une ou plusieurs des revendications 1 à 6 et/ou d'un sel compatible du point de vue physiologique de celui-ci pour la préparation d'un médicament pour la thérapie ou la prophylaxie des ulcères de l'estomac ou des zones intestinales.

12. Utilisation du composé de formule I selon une ou plusieurs des revendications 1 à 6 et/ou d'un sel compatible du point de vue physiologique de celui-ci pour la préparation d'un médicament pour la thérapie ou la prophylaxie du reflux de l'oesophage.

13. Utilisation du composé de formule I selon une ou plusieurs des revendications 1 à 6 et/ou d'un sel compatible du point de vue physiologique de celui-ci pour la préparation d'un médicament pour la thérapie ou la prophylaxie des diarrhées.

14. Utilisation du composé de formule I selon une ou plusieurs des revendications 1 à 6 et/ou d'un sel compatible du point de vue physiologique de celui-ci pour la préparation d'un médicament pour la thérapie ou la prophylaxie de tous types d'arythmies, y compris des arythmies atriales, ventriculaires et supra-ventriculaires.

15. Utilisation du composé de formule I selon une ou plusieurs des revendications 1 à 6 et/ou d'un sel compatible du point de vue physiologique de celui-ci pour la préparation d'un médicament pour la thérapie ou la prophylaxie des perturbations du rythme cardiaque qui peuvent être éliminées par augmentation du potentiel d'activation.

16. Utilisation du composé de formule I selon une ou plusieurs des revendications 1 à 6 et/ou d'un sel compatible du point de vue physiologique de celui-ci pour la préparation d'un médicament pour la thérapie ou la prophylaxie de la fibrillation atriale ou des palpitations atriales.

17. Utilisation du composé de formule I selon une ou plusieurs des revendications 1 à 6 et/ou d'un sel compatible du point de vue physiologique de celui-ci pour la préparation d'un médicament pour la thérapie ou la prophylaxie d'arythmies de retour ou pour la diminution de la mortalité cardiaque brutale à la suite de vibrations de chambre.

18. Utilisation du composé de formule I selon une ou plusieurs des revendications 1 à 6 et/ou d'un sel compatible du point de vue physiologique de celui-ci pour la préparation d'un médicament pour la thérapie de l'insuffisance cardiaque.

19. Utilisation du composé de formule I selon une ou plusieurs des revendications 1 à 2 et/ou d'un sel compatible du point de vue physiologique de celui-ci pour la préparation d'un médicament pour l'inhibition de la sécrétion de l'acide gastrique stimulé, pour la thérapie ou la prophylaxie de l'ulcère gastrique ou du domaine intestinal, du reflux de l'oesophage, des diarrhées, pour la thérapie ou la prophylaxie d'arythmies, y compris des arythmies atriales, ventriculaires et supra-ventriculaires, de la fibrillation atriale et des palpitations atriales et des arythmies de retour ou pour la diminution de la mortalité cardiaque brutale à la suite de vibrations de chambre.

20. Préparation pharmaceutique contenant une quantité d'au moins 0,1 à 10% en poids d'au moins un composé selon la formule I selon une ou plusieurs des revendications 1 à 6 et/ou un sel compatible physiologiquement de celui-ci ainsi qu'un bêtabloquant comme composé actif, avec des véhicules et des additifs acceptables du point de vue pharmaceutique.
